# EUROPEAN PATENT SPECIFICATION

(11) **EP 0 912 725 B1**
(45) Date of publication and mention of the grant of the patent: **14.07.2004**
(21) Application number: 97906876.4
(22) Date of filing: 10.01.1997
(51) Int. Cl.: C12N 9/26, C12N 1/20, C07H 21/04

(54) **GLYCOSIDASE ENZYMES**
GLYKOSIDASEENZYME
ENZYMES DE TYPE GLYCOSIDASE

(30) Priority: 11.01.1996 US 583787; 13.09.1996 US 56916 P
(43) Date of publication of application: 06.05.1999
(73) Proprietor: DIVERSA CORPORATION, San Diego, California 92121 (US)
(72) Inventor: BYLINA, Edward, J., Andalusia, PA 19020 (US); SWANSON, Ronald, V., Media, PA 19063 (US); MATHUR, Eric, J., Carlsbad, CA 92009 (US); LAM, David, E., Harbor City, CA 90710 (US)
(74) Representative: VOSSIUS & PARTNER
(86) International application number: PCT/US1997/000092
(87) International publication number: WO 1997/025417

(56) References cited:
- EP-A- 0 606 008
- EP-A- 0 687 732
- WO-A-97/20918
- WO-A-97/25417
- WO-A-97/44361
- WO-A-98/24799
- DAKHOVA O N ET AL: "CLONING AND EXPRESSION IN ESCHERICHIA COLI OF THERMOTOGA NEAPOLITANA GENES CODING FOR ENZYMES OF CARBOHYDRATE SUBSTRATE DEGRADATION" BIOCHEMICAL AND BIOPHYSICAL RESEARCH COMMUNICATIONS,US,ACADEMIC PRESS INC. ORLANDO, FL, vol. 194, no. 3, 16 August 1993 (1993-08-16), pages 1359-1364, XP002026991 ISSN: 0006-291X -& DAKHOVA ET AL.: "Thermotoga neapolitana bgA1 gene" EMBL SEQUENCE DATABASE, 1 July 1997 (1997-07-01), XP002154622 HEIDELBERG DE -& DAKHOVA ET AL.: "T. neapolitana lamA gene and bg1B gene" EMBL SEQUENCE DATABASE, 31 December 1995 (1995-12-31), XP002154623 HEIDELBERG DE
- VOORHORST W G B ET AL: "CHARACTERIZATION OF THE CELB GENE CODING FOR BETA-GLUCOSIDASE FROM THE HYPERTHERMOPHILIC ARCHAEON PYROCOCCUS FURIOSUS AND ITS EXPRESSION AND SITE-DIRECTED MUTATION IN ESCHERICHIA COLI" JOURNAL OF BACTERIOLOGY,US,WASHINGTON, DC, vol. 177, no. 24, 1 December 1995 (1995-12-01), pages 7105-7111, XP002064570 ISSN: 0021-9193
- GRAEBNITZ F ET AL: "STRUCTURE OF THE BETA-GLUCOSIDASE GENE BGLA OF CLOSTRIDIUM THERMOCELLUM. SEQUENCE ANALYSIS REVEALS A SUPERFAMILY OF CELLULASES AND BETA-GLYCOSIDASES INCLUDING HUMAN LACTASE/PHLORIZIN HYDROLASE" EUROPEAN JOURNAL OF BIOCHEMISTRY,DE,BERLIN, vol. 200, no. 2, 1 September 1991 (1991-09-01), pages 301-309, XP000874574 ISSN: 0014-2956
- CANGANELLA F ET AL: "CHARACTERIZATION OF AMYLOLYTIC AND PULLULYTIC ENZYMES FROM THERMOPHILIC ARCHAEA AND FROM A NEW FERVIDOBACTERIUM SPECIES" APPLIED MICROBIOLOGY AND BIOTECHNOLOGY,DE,SPRINGER VERLAG, BERLIN, vol. 42, no. 2/03, 1994, pages 239-245, XP000602919 ISSN: 0175-7598
- BAUER ET AL.: "Comparison of a beta-glucosidase and a beta-mannosidase from hyperthermophilic archaeon Pyrococcus furiosus" THE JOURNAL OF BIOLOGICAL CHEMISTRY, vol. 271, no. 39, 27 September 1996 (1996-09-27), pages 23749-23755, XP002154624 -& BAUER ET AL.: "Pyrococcus furiosus beta mannosidase (bmnA) gene, complete ccds" EMBL SEQUENCE DATABASE, 2 July 1996 (1996-07-02), XP002154625 HEIDELBERG DE -& BAUER ET AL.: "Beta-mannosidase" EMBL SEQUENCE DATABASE, 1 November 1996 (1996-11-01), XP002154626 HEIDELBERG DE
- J. BACTERIOLOGY, December 1995, Vol. 177, No. 24, VOORHORST et al., "Characterization of the CelB Gene Coding for Beta-glucosidase from the Hyperthermophilic Archaeon Pyrococcus Furiosus and Its Expression and Site-directed Mutation in Escherichia Coli", pages 7105-7111, XP002064570.
- DATABASE CAPLUS on STN, CAS, (Columbus, Ohio, USA), AN 1996:106914, KENGEN et al., "An Extremely Thermostable .beta.-Glucosidase from the Hyperthermophilic Archaeon Pyrococcus Furiosus; a Comparison With Other Glycosidases", XP002943860 & BIOCATALYSIS 1994, Vol. 11, No. 2, pages 79-88.
- J. BIOL. CHEM., 27 September 1996, Vol. 271, No. 39, BAUER et al., "Comparison of Beta-glucosidase and Beta-mannosidase from the Hyperthermophilic Archaeon Pyrococcus Furiosus", pages 23749-23755, XP002154624.

## Description

This invention relates to a newly identified polynucleotide, polypeptide encoded by such polynucleotide, the use of such polynucleotides and polypeptide, as well as the production and isolation of such polynucleotides and polypeptides. More particularly, the polynucleotides and polypeptides of the present invention has been putatively identified as a pullalanase.

The glycosidic bond of β-galactosides can be cleaved by different classes of enzymes: (i) phospho-β-galactosidases (EC3.2.1.85) are specific for a phosphorylated substrate generated via phosphoenolpyruvate phosphotransferase system (PTS)-dependent uptake; (ii) typical β-galactosidases (EC 3.2.1.23), represented by the *Escherichia coli* LacZ enzyme, which are relatively specific for β-galactosides; and (iii) β-glucosidases (EC 3.2.1.21) such as the enzymes of *Agrobacterium faecalis, Clostridium thermocellum, Pyrococcus furiosus* or *Sulfolobus solfataricus* (Day, A.G. and Withers, S.G., (1986) Purification and characterization of a β-glucosidase from *Alcaligenes faecalis.* Can. J. Biochem. Cell. Biol. 64, 914-922; Kengen, S.W.M., et al. (1993) Eur. J. Biochem., 213, 305-312; Ait, N., Cruezet, N. and Cattaneo, J. (1982) Properties of β-glucosidase purified from *Clostridium thermocellum*. J. Gen. Microbiol. 128, 569-577; Grogan, D.W. (1991) Evidence that β-galactosidase of *Sulfolobus solfataricus* is only one of several activities of a thermostable β-D-glycodiase. Appl. Environ. Microbiol. 57, 1644-1649). Members of the latter group, although highly specific with respect to the β-anomeric configuration of the glycosidic linkage, often display a rather relaxed substrate specificity and hydrolyse β-glucosides as well as β-fucosides and β-galactosides.

Generally, α-galactosidases are enzymes that catalyze the hydrolysis of galactose groups on a polysaccaride backbone or hydrolyze the cleavage of di- or oligosaccharides comprising galactose.

Generally, β-mannanases are enzymes that catalyze the hydrolysis of mannose groups internally on a polysaccaride backbone or hydrolyze the cleavage of di- or oligosaccaharides comprising mannose groups. β-mannosidases hydrolyze non-reducing, terminal mannose residues on a mannose-containing polysaccharide and the cleavage of di- or oligosaccaharides comprising mannose groups.

Guar gum is a branched galactomannan polysaccharide composed of β-1,4 linked mannose backbone with α-1,6 linked galactose sidechains. The enzymes required for the degradation of guar are β-mannanase, β-mannosidase and α-galactosidase. β-mannanase hydrolyses the mannose backbone internally and β-mannosidase hydrolyses non-reducing, terminal mannose residues. α-galactosidase hydrolyses α-linked galactose groups.

Galactomannan polysaccharides and the enzymes that degrade them have a variety of applications. Guar is commonly used as a thickening agent in food and is utilized in hydraulic fracturing in oil and gas recovery. Consequently, galactomannanases are industrially relevant for the degradation and modification of guar. Furthermore, a need exists for thermostable galactomannases that are active in extreme conditions associated with drilling and well stimulation.

There are other applications for these enzymes in various industries, such as in the beet sugar industry. 20-30% of the domestic U.S. sucrose consumption is sucrose from sugar beets. Raw beet sugar can contain a small amount of raffinose when the sugar beets are stored before processing and rotting begins to set in. Raffinose inhibits the crystallization of sucrose and also constitutes a hidden quantity of sucrose. Thus, there is merit to eliminating raffinose from raw beet sugar. α-Galactosidase has also been used as a digestive aid to break down raffinose, stachyose, and verbascose in such foods as beans and other gassy foods.

β-Galactosidases which are active and stable at high temperatures appear to be superior enzymes for the production of lactose-free dietary milk products (Chaplin, M.F. and Bucke, C. (1990) In: Enzyme Technology, pp. 159-160, Cambridge University Press, Cambridge, UK). Also, several studies have demonstrated the applicability of β-galactosidases to the enzymatic synthesis of oligosaccharides via transglycosylation reactions (Nilsson, K.G.I. (1988) Enzymatic synthesis of oligosaccharides. Trends Biotechnol. 6, 156-264; Cote, G.L. and Tao, B.Y. (1990) Oligosaccharide synthesis by enzymatic transglycosylation. Glycoconjugate J. 7, 145-162). Despite the commercial potential, only a few β-galactosidases of thermophiles have been characterized so far. Two genes reported are β-galactoside-cleaving enzymes of the hyperthermophilic bacterium *Thermotoga maritima*, one of the most thermophilic organotrophic eubacteria described to date (Huber, R., Langworthy, T.A., König, H., Thomm, M., Woese, C.R., Sleytr, U.B. and Stetter, K.O. (1986) *T*. *martima* sp. nov. represents a new genus of unique extremely thermophilic eubacteria growing up to 90°C, Arch. Microbiol. 144, 324-333) one of the most thermophilic organotrophic eubacteria described to date. The gene products have been identified as a β-galactosidase and a β-glucosidase.

Pullulanase is well known as a debranching enzyme of pullulan and starch. The enzyme hydrolyzes α-1,6-glucosidic linkages on these polymers. Starch degradation for th eproduction or sweeteners (glucose or maltose) is a very important industrial application of this enzyme. The degradation of starch is developed in two stages. The first stage involves the liquefaction of the substrate with α-amylase, and the second stage, or saccharification stage, is performed by β-amylase with pullalanase added as a debranching enzyme, to obtain better yields.

Endoglucanases can be used in a variety of industrial applications. For instance, the endoglucanases of the present invention can hydrolyze the internal β-1,4-glycosidic bonds in cellulose, which may be used for the conversion of plant biomass into fuels and chemicals. Endoglucanases also have applications in detergent formulations, the textile industry, in animal feed, in waste treatment, and in the fruit juice and brewing industry for th eclarification and extraction of juices.

The polynucleotides and polypeptides of the present invention have been identified as pullalanases as a result of its enzymatic activity.

In accordance with one aspect of the present invention, there are provided novel enzymes, as well as active fragments, analogs and derivatives thereof.

In accordance with another aspect of the present invention, there are provided isolated nucleic acid molecules encoding the enzymes of the present invention including mRNAs, cDNAs, genomic DNAs as well as active analogs and fragments of such enzymes.

In accordance with another aspect of the present invention there are provided isolated nucleic acid molecules encoding mature polypeptides expressed by the DNA contained in ATCC Deposit No. 97379.

In accordance with yet a further aspect of the present invention, there is provided a process for producing such polypeptides by recombinant techniques comprising culturing recombinant prokaryotic and/or eukaryotic host cells, containing a nucleic acid sequence of the present invention, under conditions promoting expression of said enzymes and subsequent recovery of said enzymes.

In accordance with yet a further aspect of the present invention, there is provided a process for utilizing such enzymes, or polynucleotides encoding such enzymes for hydrolyzing lactose to galactose and glucose for use in the food processing industry, the pharmaceutical industry, for example, to treat intolerance to lactose, as a diagnostic reporter molecule, in corn wet milling, in the fruit juice industry, in baking, in the textile industry and in the detergent industry.

In accordance with yet a further aspect of the present invention, there is provided a process for utilizing such enzymes for hydrolyzing guar gum (a galactomannan polysaccharide) to remove non-reducing terminal mannose residues. Further polysaccharides such as galactomannan and the enzymes according to the invention that degrade them have a varitey of applications. Guar gum is commonly used as a thickening agent in food and also is utilized in hydraulic fracturing in oil and gas recovery. Consequently, mannanases are industrially relevant for the degradation and modification of guar gums. Furthermore, a need exists for thermostable mannases that are active in extreme conditions associated with drilling and well stimulation.

In accordance with yet a further aspect of the present invention, there are also provided nucleic acid probes comprising nucleic acid molecules of sufficient length to specifically hybridize to a nucleic acid sequence of the present invention.

In accordance with yet a further aspect of the present invention, there is provided a process for utilizing such enzymes, or polynucleotides encoding such enzymes, for *in vitro* purposes related to scientific research, for example, to generate probes for identifying similar sequences which might encode similar enzymes from other organisms by using certain regions, *i.e.*, conserved sequence regions, of the nucleotide sequence.

These and other aspects of the present invention should be apparent to those skilled in the art from the teachings herein.

### Brief Description of the Drawings

Figure 1 is an illustration of the full-length DNA and corresponding deduced amino acid sequence of M11TL of the present invention. Sequencing was performed using a 378 automated DNA sequencer for all sequences of the present invention (Applied Biosystems, Inc.).

Figure 2 is an illustration of the full-length DNA and corresponding deduced amino acid sequence of OC1/4V-33B/G.

Figure 3 is an illustration of the full-length DNA and corresponding deduced amino acid sequence of F1-12G.

Figure 4 are illustrations of the full-length DNA and corresponding deduced amino acid sequence of 9N2-31B/G.

Figure 5 are illustrations of the full-length DNA and corresponding deduced amino acid sequence of MSB8-6G.

Figure 6 are illustrations of the full-length DNA and corresponding deduced amino acid sequence of AEDII12RA-18B/G.

Figure 7 is an illustration of the full-length DNA and corresponding deduced amino acid sequence of GC74-22G.

Figure 8 is an illustration of the full-length DNA and corresponding deduced amino acid sequence of VC1-7G1.

Figure 9 is an illustration of the full-length DNA and corresponding deduced amino acid sequence of 37GP1.

Figure 10 is an illustration of the full-length DNA and corresponding deduced amino acid sequence of 6GC2.

Figure 11 is an illustration of the full-length DNA and corresponding deduced amino acid sequence of 6GP2.

Figure 12 is an illustration of the full-length DNA and corresponding deduced amino acid sequence of 63GB1.

Figure 13 is an illustration of the full-length DNA and corresponding deduced amino acid sequence of OC1/4V.

Figure 14 is an illustration of the full-length DNA and corresponding deduced amino acid sequence of 6GP3.

### Definitions

The term "gene" means the segment of DNA involved in producing a polypeptide chain; it includes regions preceding and following the coding region (leader and trailer) as well as intervening sequences (introns) between individual coding segments (exons).

A coding sequence is "operably linked to" another coding sequence when RNA polymerase will transcribe the two coding sequences into a single mRNA, which is then translated into a single polypeptide having amino acids derived from both coding sequences. The coding sequences need not be contiguous to one another so long as the expressed sequences ultimately process to produce the desired protein.

"Recombinant" enzymes refer to enzymes produced by recombinant DNA techniques; i.e., produced from cells transformed by an exogenous DNA construct encoding the desired enzyme. "Synthetic" enzymes are those prepared by chemical synthesis.

A DNA "coding sequence of" or a "nucleotide sequence encoding" a particular enzyme, is a DNA sequence which is transcribed and translated into an enzyme when placed under the control of appropriate regulatory sequences.

### Summary of the Invention

In accordance with an aspect of the present invention, there are provided isolated nucleic acid (polynucleotide) SEQ ID 14 which encode for the mature enzyme having the deduced amino acid sequences of SEQ ID 28.

In accordance with another aspect of the present invention, there are provided isolated polynucleotides encoding the enzymes of the present invention. The deposited material is a mixture of genomic clones comprising DNA encoding an enzyme of the present invention. Each genomic clone comprising the respective DNA has been inserted into a pBluescript vector (Stratagene, La Jolla, CA). The deposit has been deposited with the American Type Culture Collection, 12301 Parklawn Drive, Rockville, Maryland 20852, USA, on December 13, 1995 and assigned ATCC Deposit No. 97379.

The deposit(s) have been made under the terms of the Budapest Treaty on the International Recognition of the deposit of micro-organisms for purposes of patent procedure. The strains will be irrevocably and without restriction or condition released to the public upon the issuance of a patent. These deposits are provided merely as convenience to those of skill in the art and are not an admission that a deposit be required under 35 U.S.C. §112. The sequences of the polynucleotides contained in the deposited materials, as well as the amino acid sequences of the polypeptides encoded thereby, are controlling in the event of any conflict with any description of sequences herein. A license may be required to make, use or sell the deposited materials, and no such license is hereby granted.

### Detailed Description of the Invention

The polynucleotides of this invention were originally recovered from genomic gene libraries derived from the following organisms:

Ml1TL is a new species of *Desulfurococcus* isolated from Diamond Pool in Yellowstone National Park. The organism grows optimally at 85-88°C, pH 7.0 in a low salt medium containing yeast extract, peptone, and gelatin as substrates with a N₂/CO₂ gas phase.

OC1/4V is from the genus *Thermotoga*. The organism was isolated from Yellowstone National Park. It grows optimally at 75°C in a low salt medium with cellulose as a substrate and N₂ in gas phase.

*Pyrococcus furiosus* VC1 is from the genus *Pyrococcus*. VC1 was isolated from Vulcano, Italy. It grows optimally at 100°C in a high salt medium (marine) containing elemental sulfur, yeast extract, peptone and starch as substrates and N₂ in gas phase.

*Staphylothermus marinus* F1 is a from the genus *Staphylothermus*. F1 was isolated from Vulcano, Italy. It grows optimally at 85°C, pH 6.5 in high salt medium (marine) containing elemental sulfur and yeast extract as substrates and N₂ in gas phase.

*Thermococcus* 9N-2 is from the genus *Thermococcus* 9N-2 was isolated from diffuse vent fluid in the East Pacific Rise. It is a strict anaerobe that grows optimally at 87°C.

*Thermotoga maritima* MSB8 is from the genus *Thermotogo*, and was isolated from Vulcano, Italy. MSB8 grows optimally at 85°C, pH 6.5 in a high salt medium (marine) containing starch and yeast extract as substrates and N₂ in gas phase.

*Thermococcus alcaliphilus* AEDII12RA is from the genus *Thermococcus*. AEDII12RA grows optimally at 85°C, pH 9.5 in a high salt medium (marine) containing polysulfides and yeast extract as substrates and N₂ in gas phase.

*Thermococcus chitonophagus* GC74 is from the genus *Thermococcus*. GC74 grows optimally at 85°C, pH 6.0 in a high salt medium (marine) containing chitin, meat extract, elemental sulfur and yeast extract as substrates and N₂ in gas phase. AEPII 1a grows optimally at 85°C at pH 6.5 in marine medium under anaerobic conditions. It has many substrates.

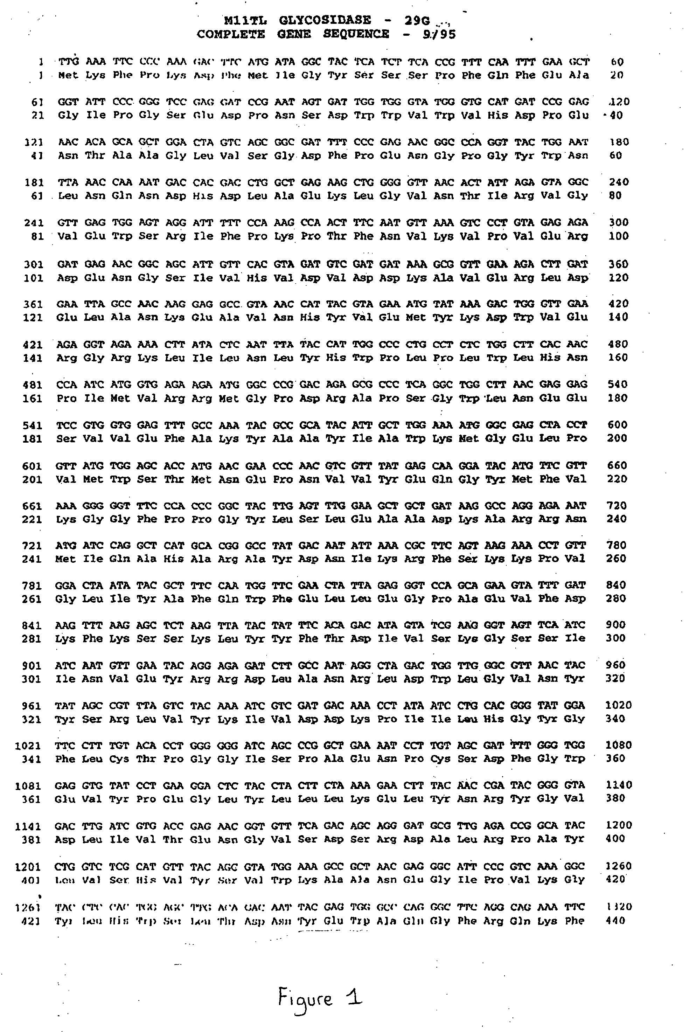

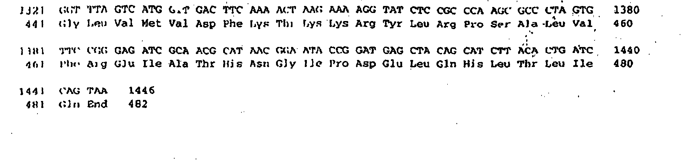

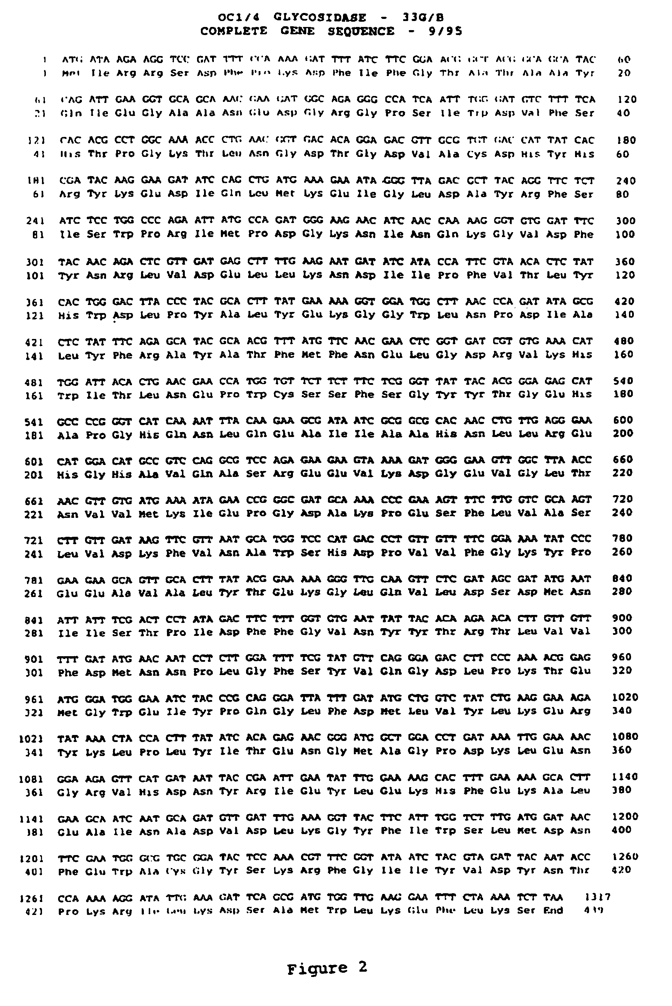

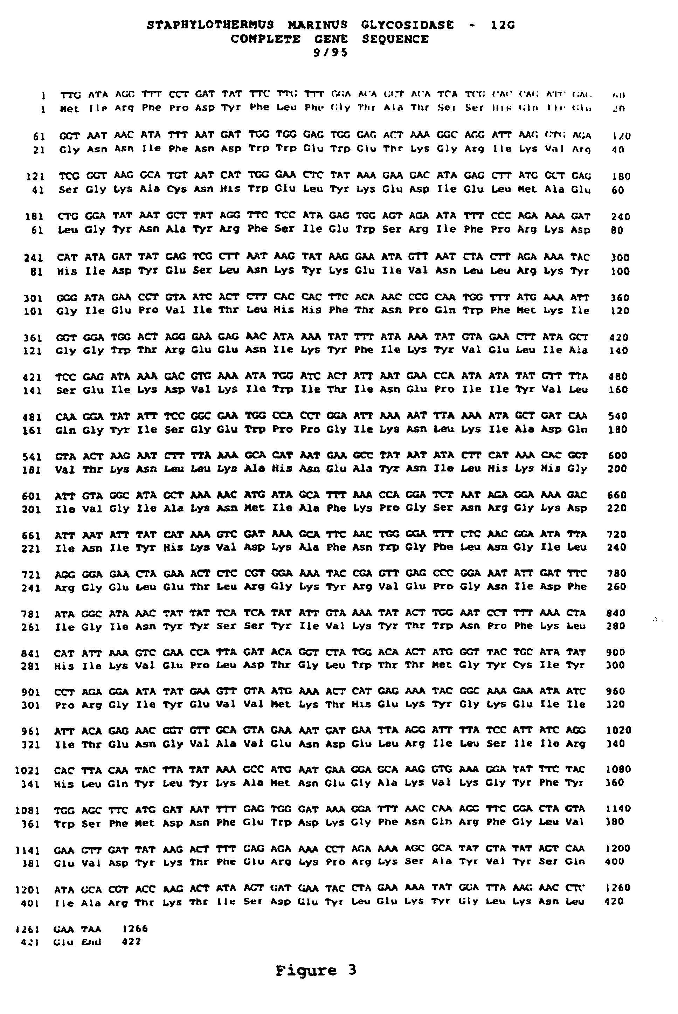

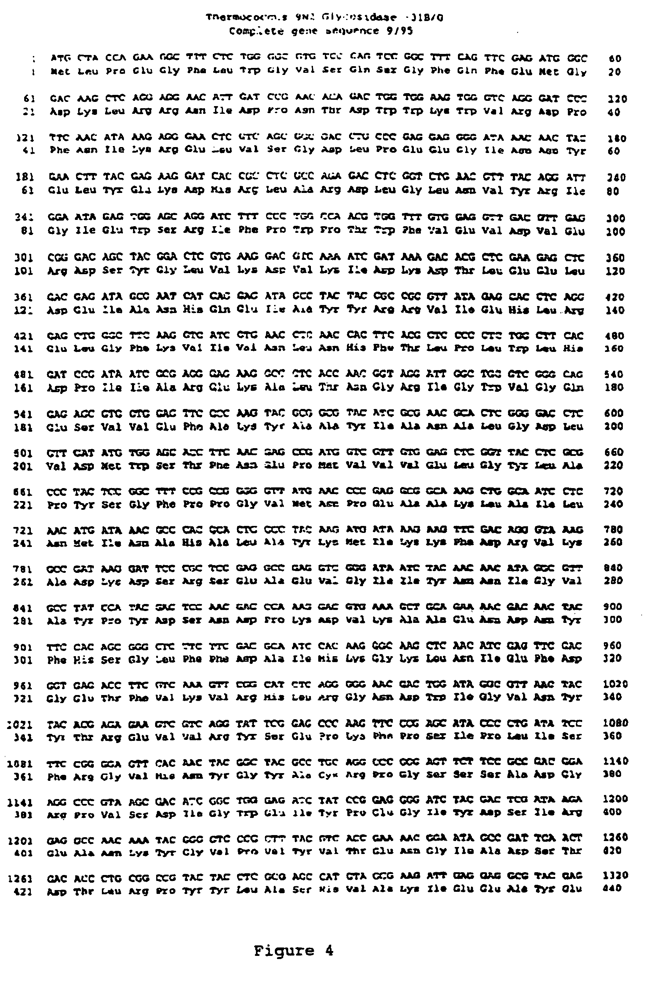

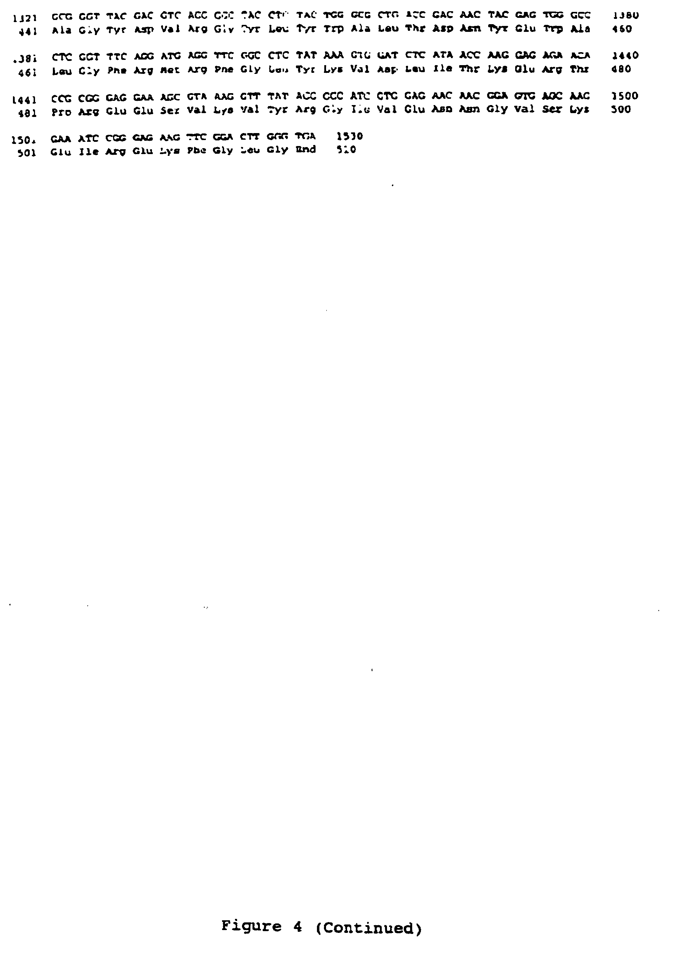

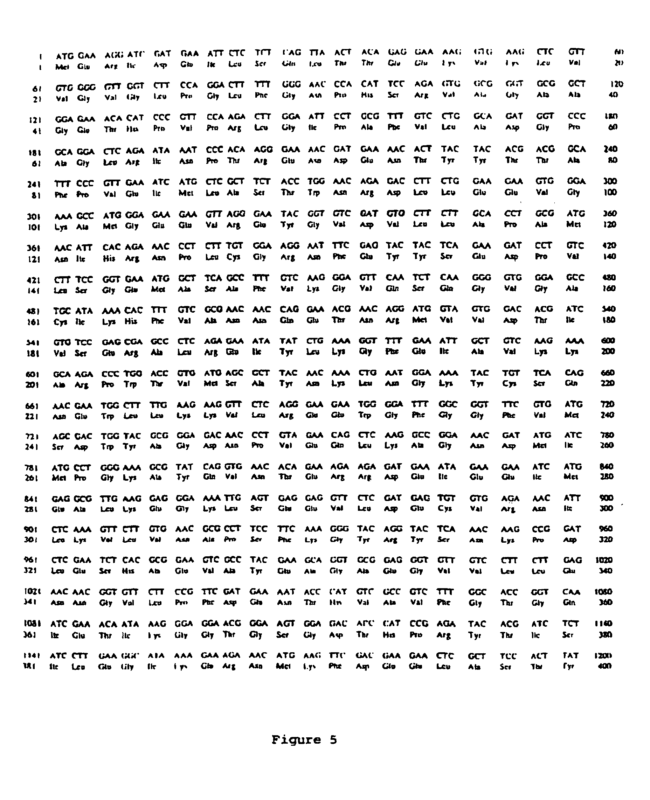

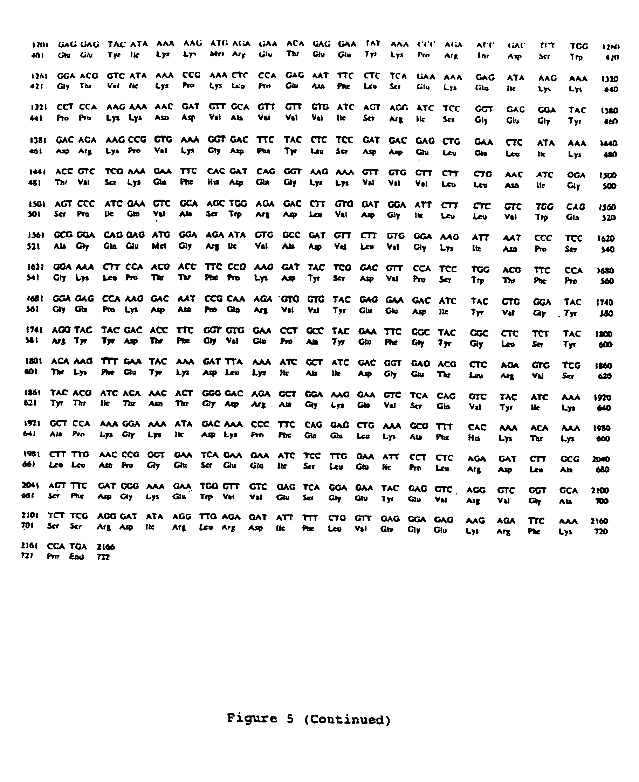

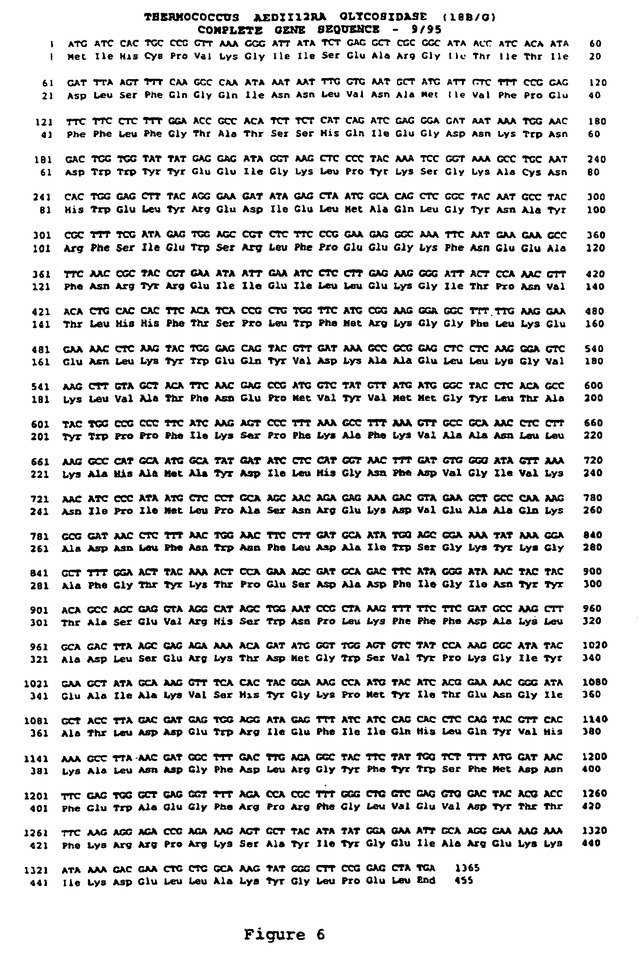

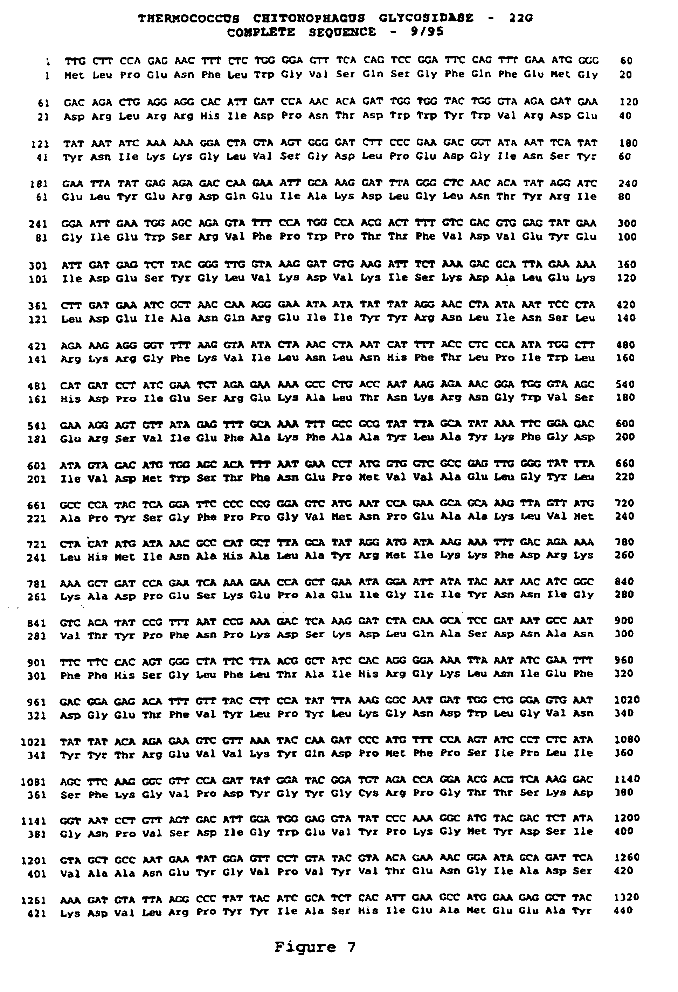

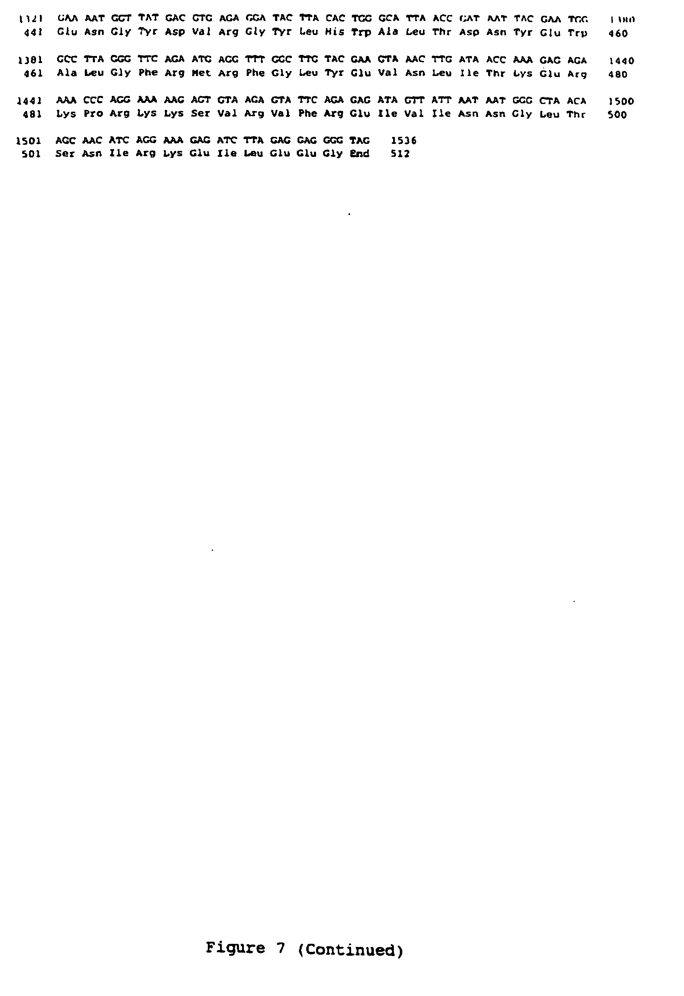

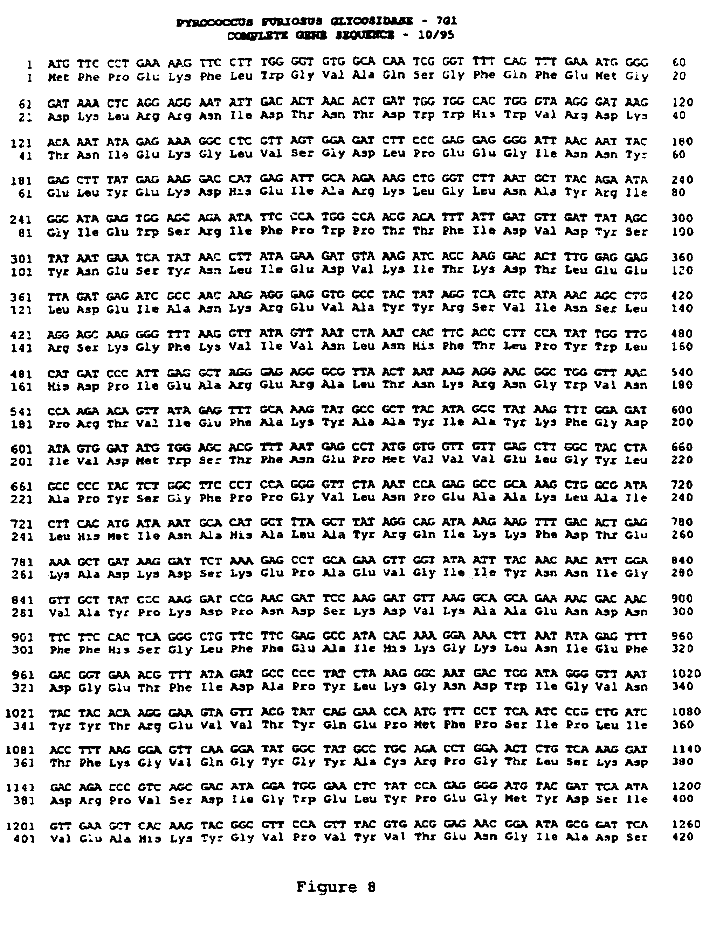

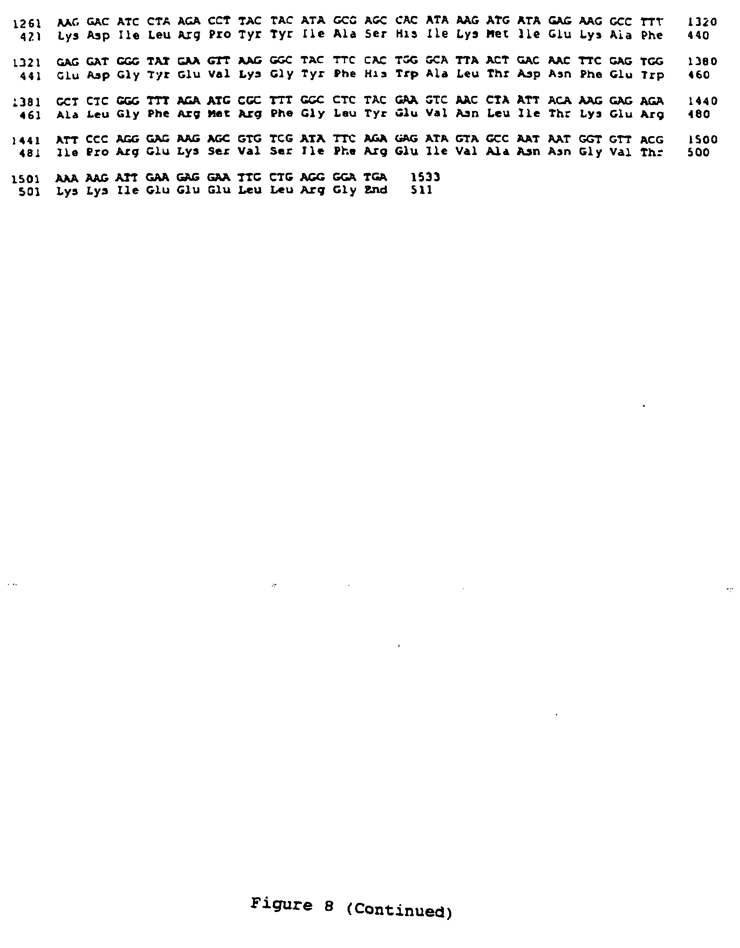

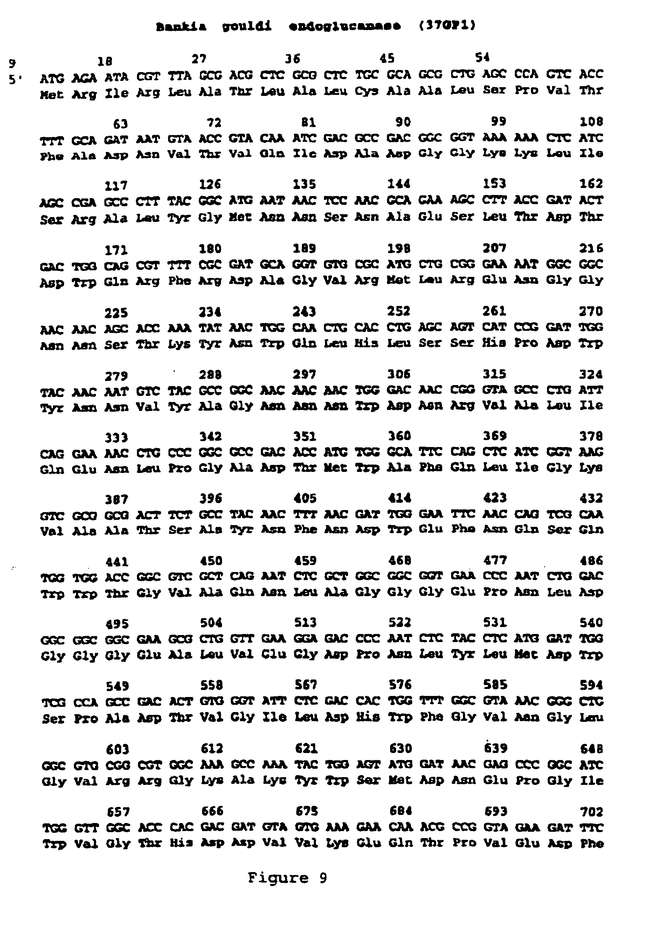

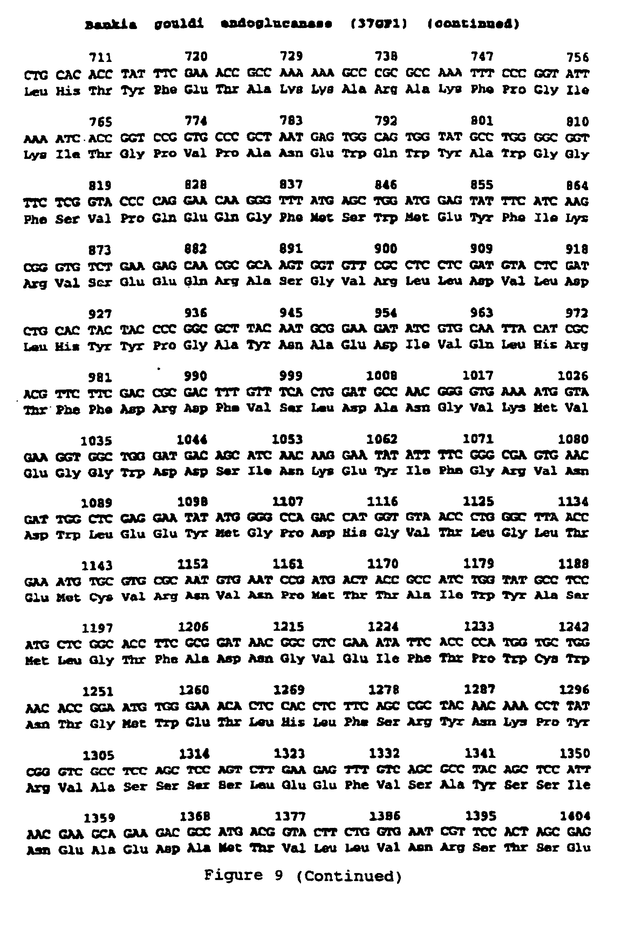

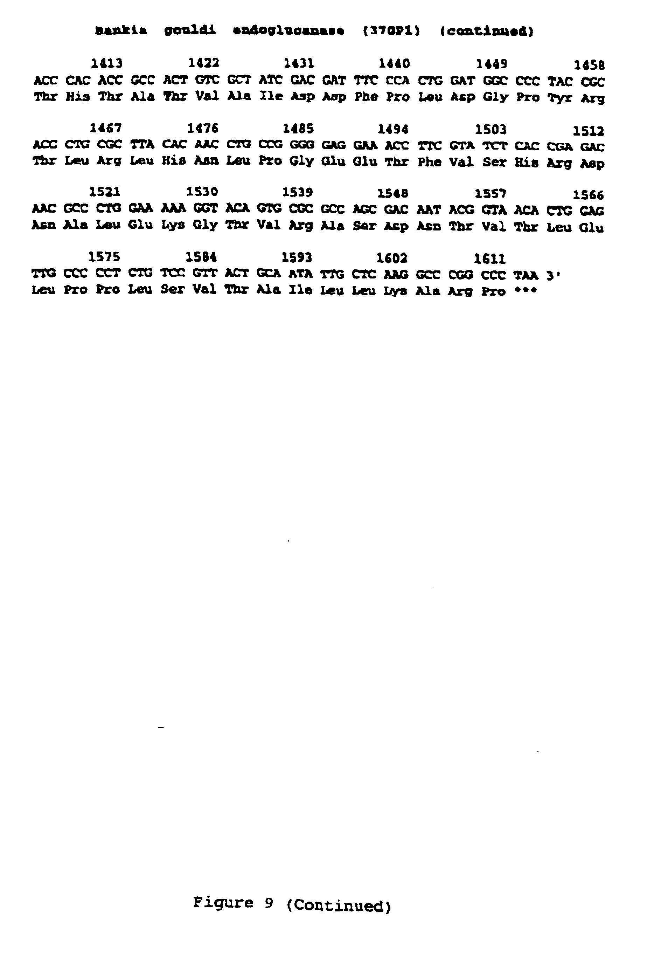

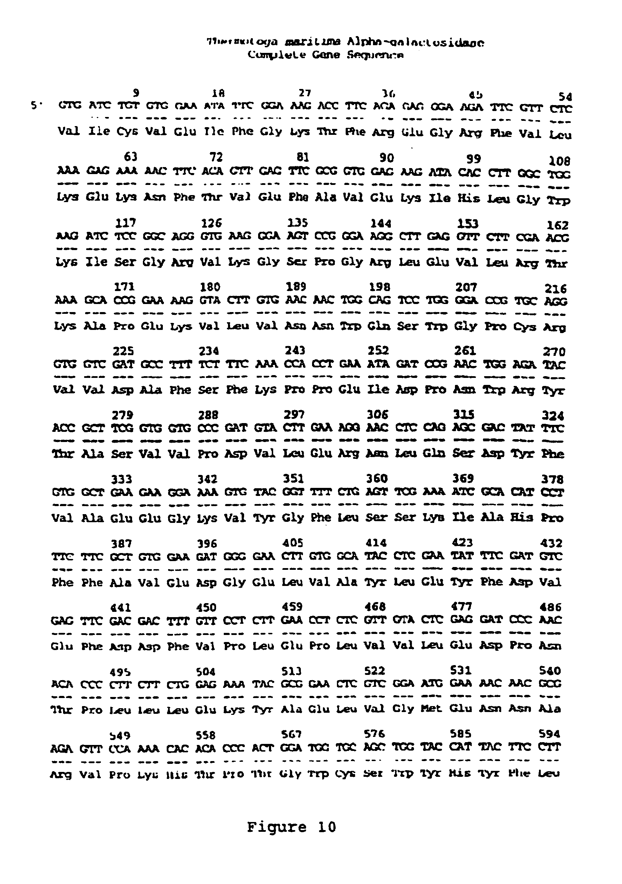

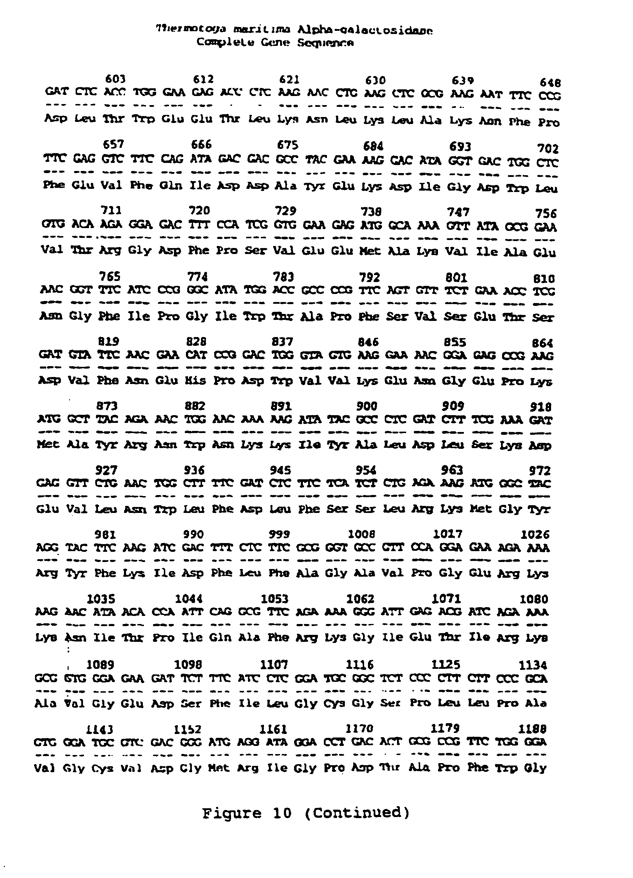

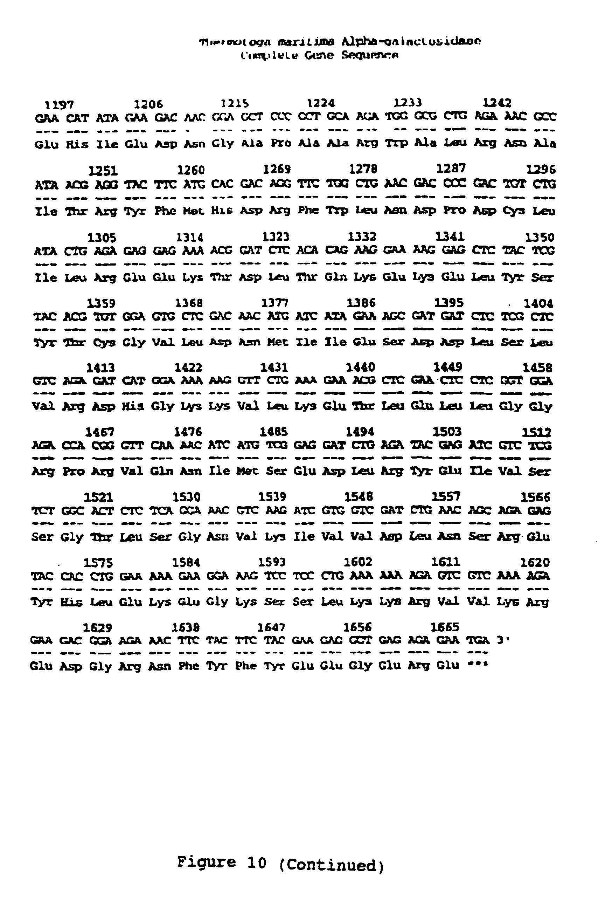

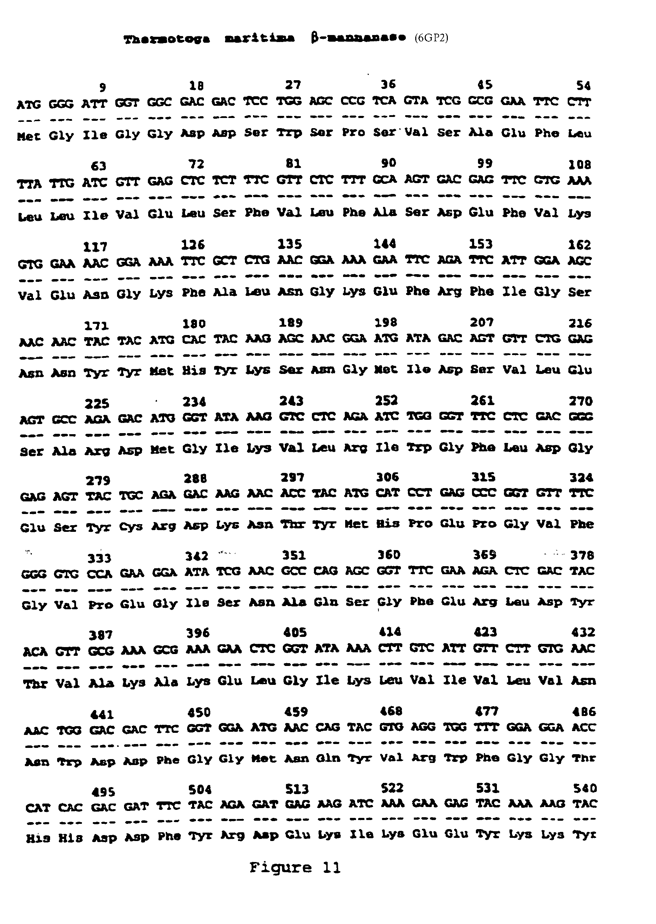

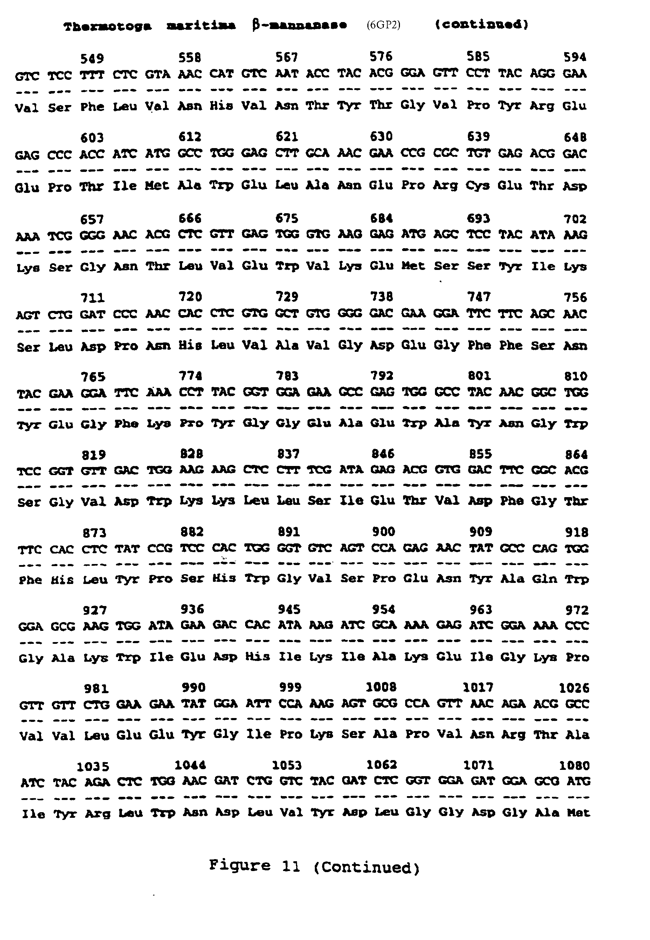

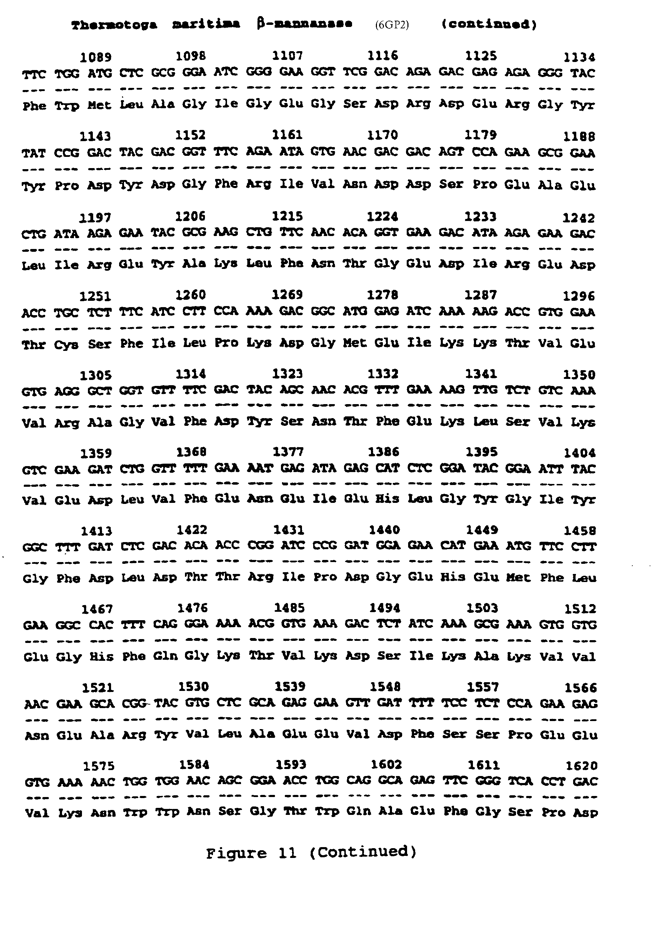

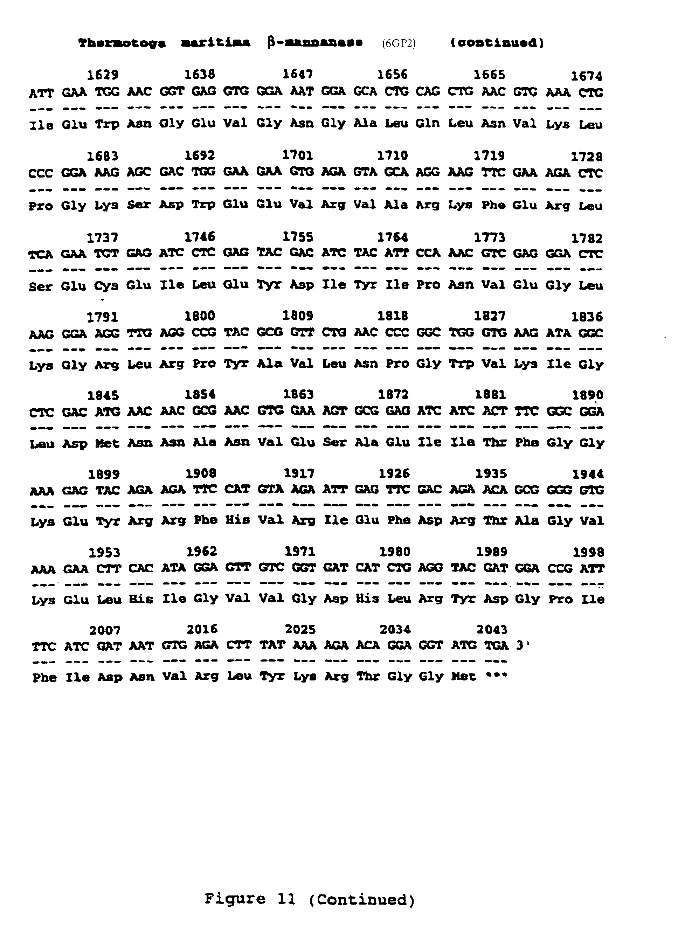

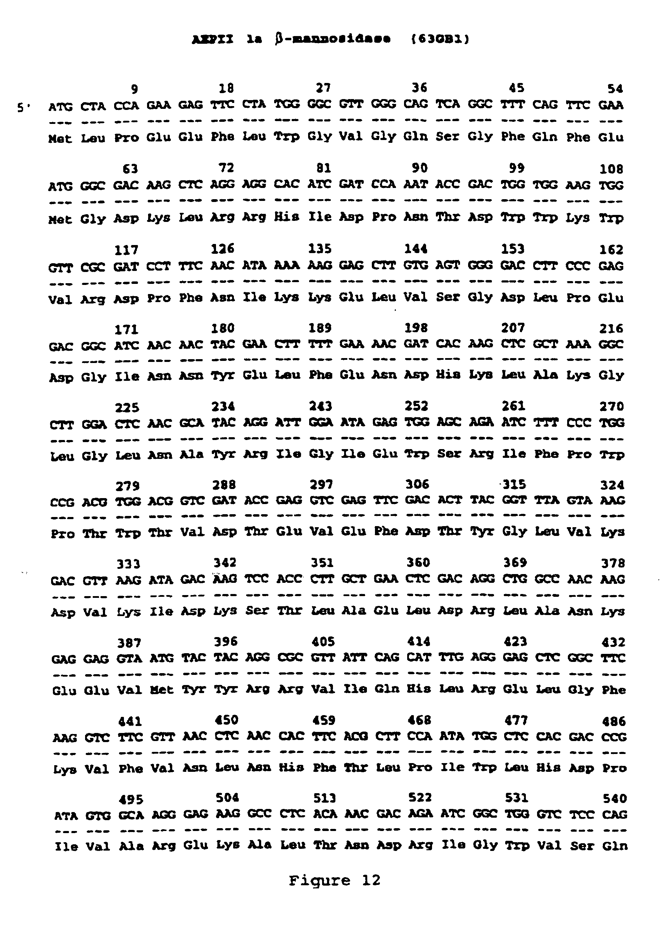

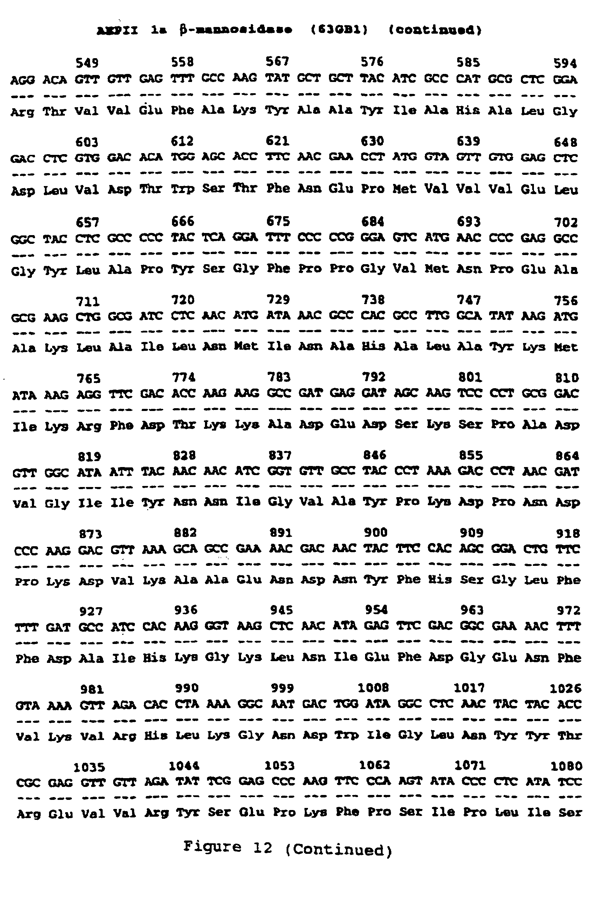

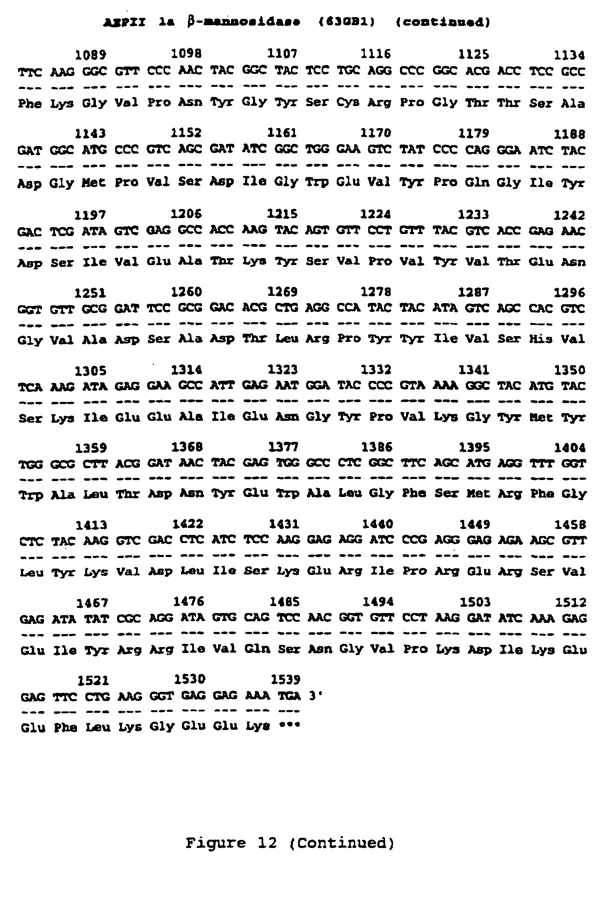

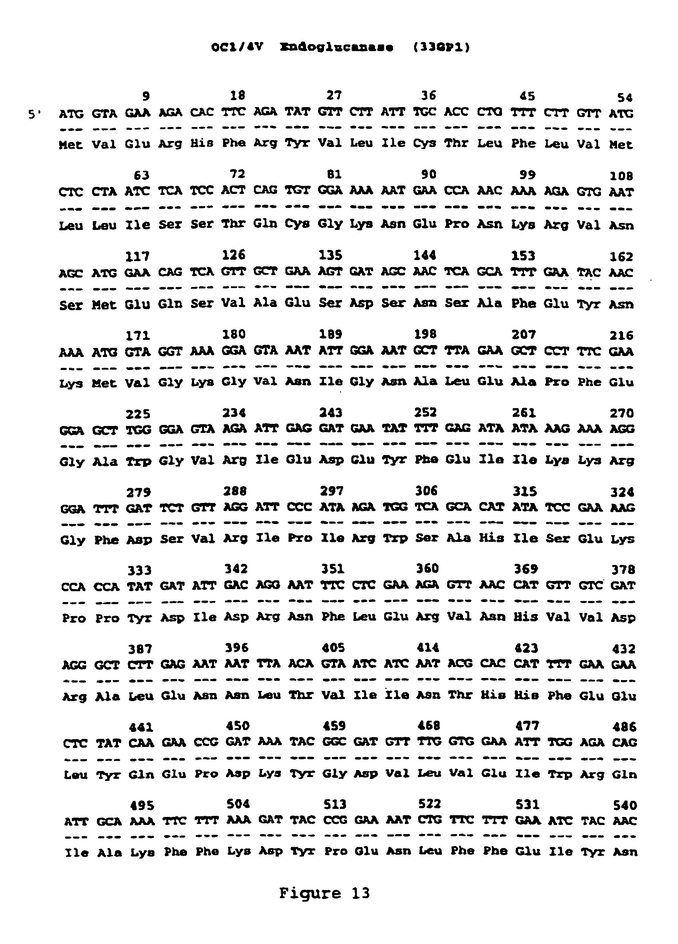

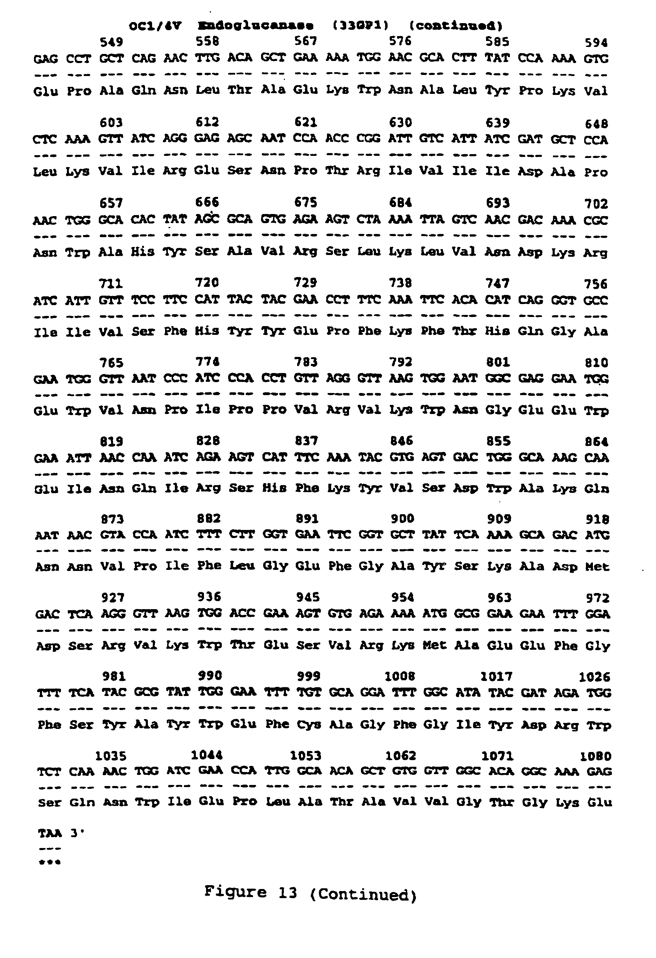

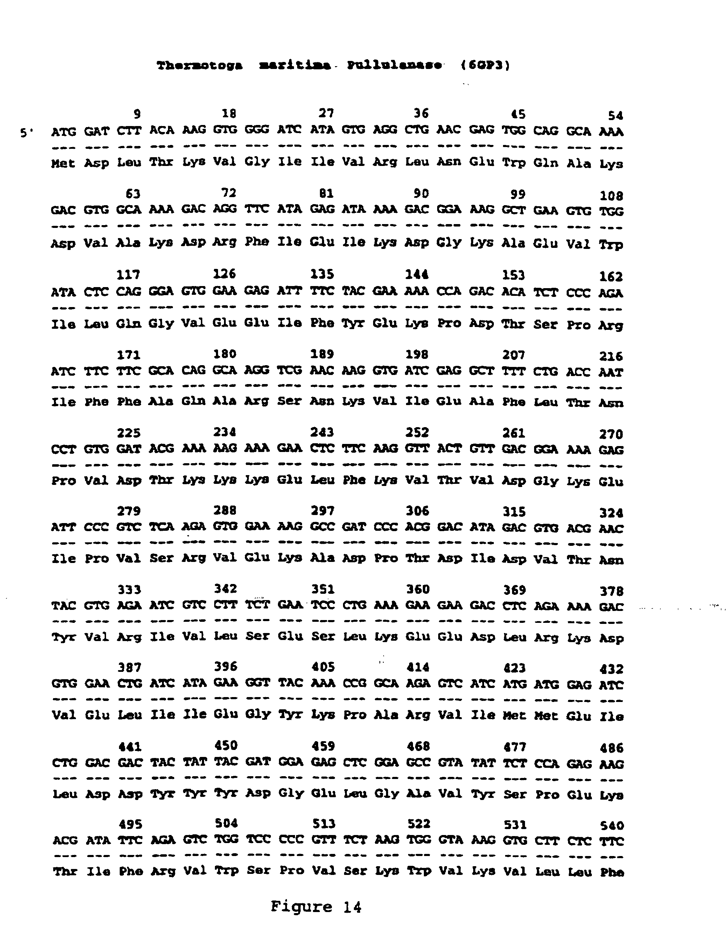

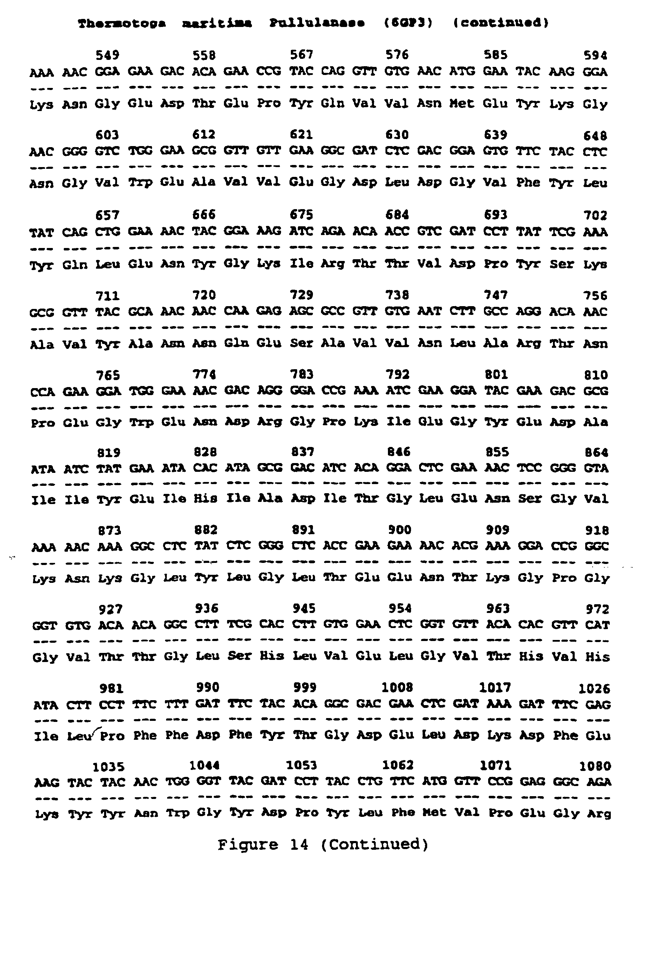

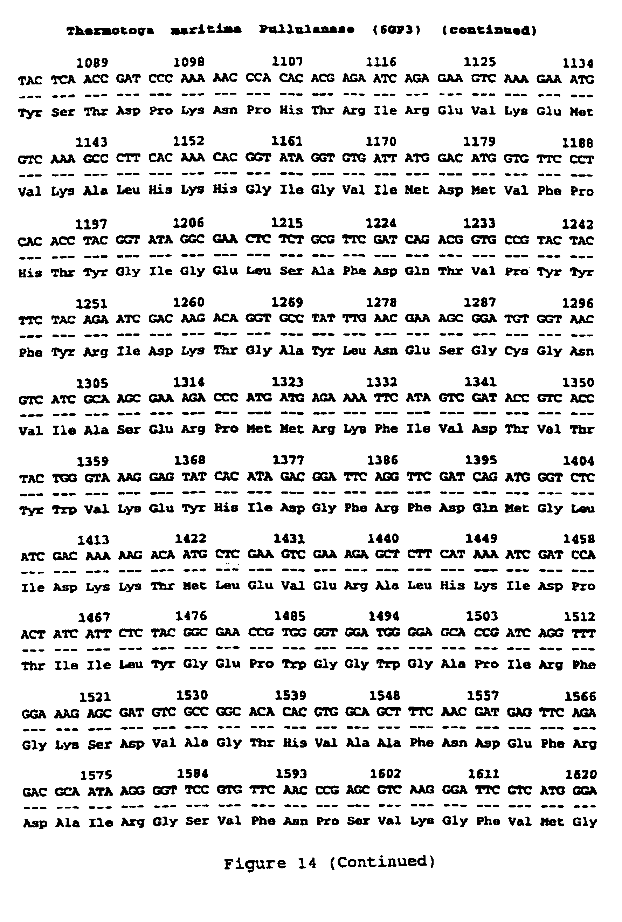

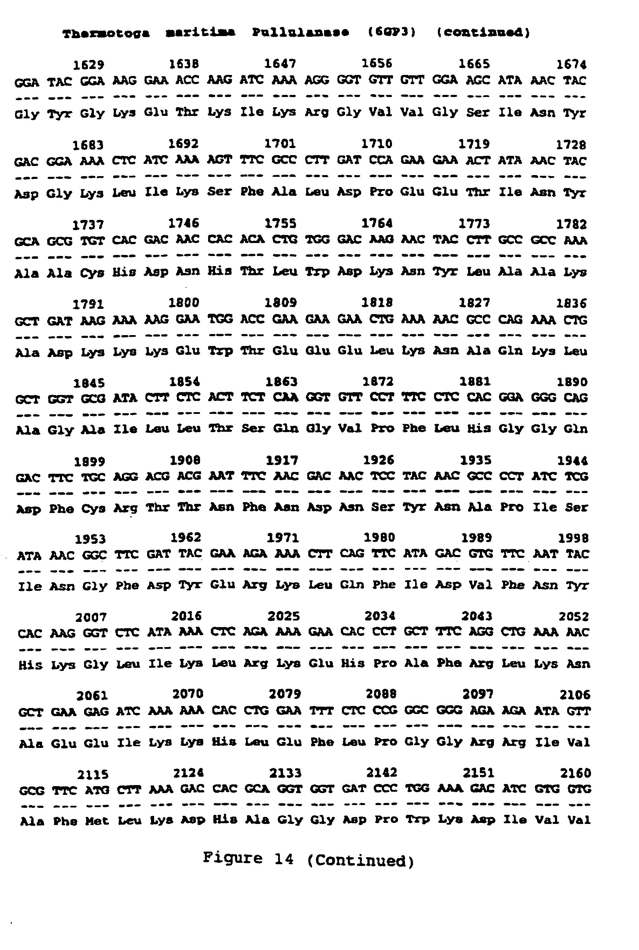

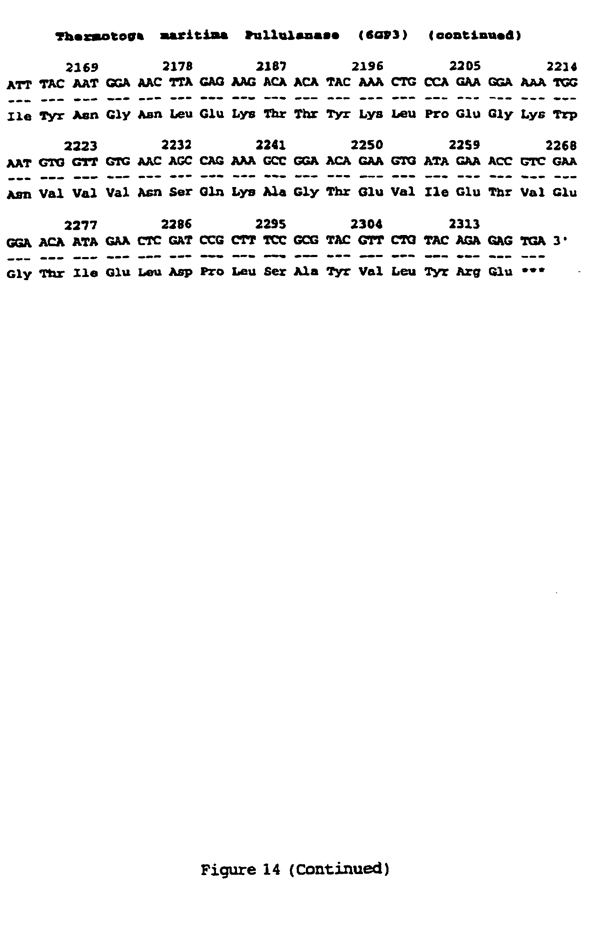

Accordingly, the polynucleotides and enzymes encoded thereby are identified by the organism from which they were isolated, and are sometimes hereinafter referred to as "M11TL" (Figure 1 and SEQ ID NOS:1 and 15), "OC1/4V-33B/G" (Figure 2 and SEQ ID NOS:2 and 16), "F1-12G" (Figure 3 and SEQ ID NOS:3 and 17), "9N2-31B/G" (Figure 4 and SEQ ID NOS:4 and 18), "MSB8" (Figure 5 and SEQ ID NOS:5 and 19), "AEDII12RA-18B/G" (Figure 6 and SEQ ID NOS:6 and 20), "GC74-22G" (Figure 7 and SEQ ID NOS:7 and 21), "VC1-7G1" (Figure 8 and SEQ ID NOS:8 and 22), "37GP1" (Figure 9 and SEQ ID NOS: 9 and 23), "6GC2" (Figure 10 and SEQ ID NOS: 10 and 24), "6GP2" (Figure 11 and SEQ ID NOS:11 and 25), "AEPII 1a" (Figure 12 and SEQ ID NOS:12 and 26), "OC1/4V" (Figure 13 and SEQ ID NOS:13 and 27), and "6GP3" (Figure 14 and SEQ ID NOS:14 and 28).

The polynucleotides and polypeptides show identity at the nucleotide and protein level to known genes and proteins encoded thereby as shown in Table 1.

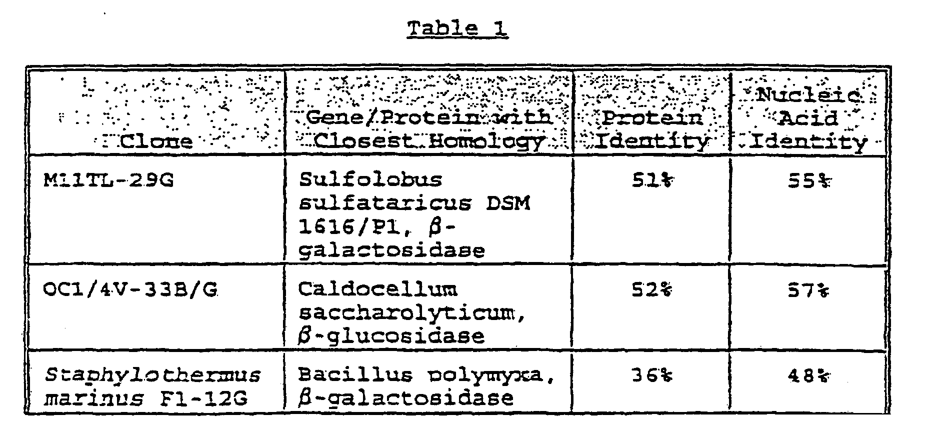

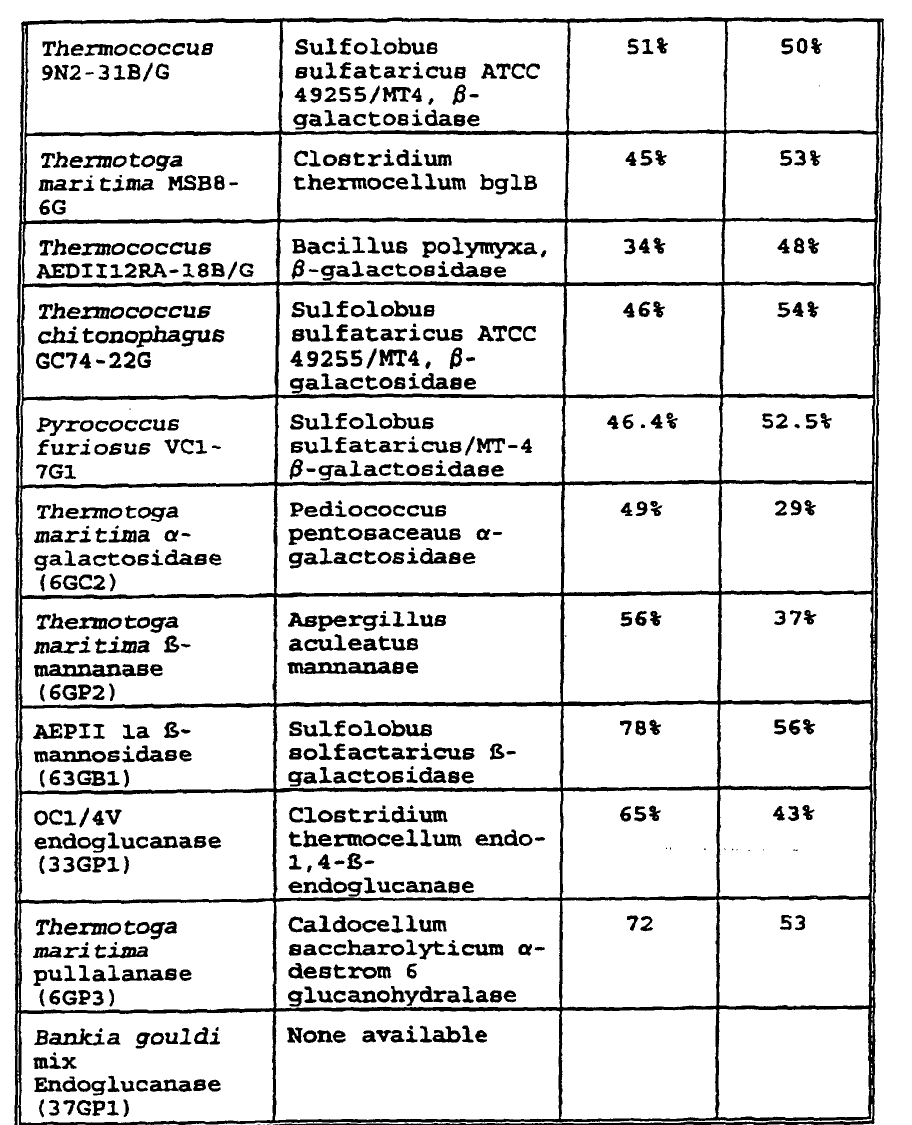

The polynucleotides and enzymes show homology to each other as shown in Table 2.

**Table 2**

| Clone | Gene/Protein with Closest Homology | Protein Identity | Nucleic Acid Identity |
|---|---|---|---|
| *Staphylothermus marinus* F1-12G | *Thermococcus* AEDII12RA-18B/G, β-galactosidase, glucosidase | 55% | 57% |
| *Thermococcus* 9N2-31B/G | *Thermococcus chitonophagus* GC74-22G-glucosidase | 74% | 66% |
| *Pyrococcus furiosus* VC1-7G1 | *Pyrococcus furiosus* VC1-7B/G β-galactosidase | 46.4% | 54% |

All the clones identified in Tables 1 and 2 encode polypeptides which have α-glycosidase or β-glycosidase activity.

This invention, in addition to the isolated nucleic acid molecules encoding the enzymes of the present invention, also provide substantially similar sequences. Isolated nucleic acid sequences are substantially similar if: (i) they are capable of hybridizing under conditions hereinafter described, to the polynucleotides of SEQ ID NOS:14; (ii) or they encode DNA sequences which are degenerate to the polynucleotides of SEQ ID NOS:14. Degenerate DNA sequences encode the amino acid sequences of SEQ ID NOS:28 but have variations in the nucleotide coding sequences. As used herein, substantially similar refers to the sequences having similar identity to the sequences of the instant invention. The nucleotide sequences that are substantially the same can be identified by hybridization or by sequence comparison. Enzyme sequences that are substantially the same can be identified by one or more of the following: proteolytic digestion, gel electrophoresis and/or microsequencing.

One means for isolating the nucleic acid molecules encoding the enzymes of the present invention is to probe a gene library with a natural or artificially designed probe using art recognized procedures (see, for example: Current protocols in Molecular Biology, Ausubel F.M. *et al*. (EDS.) Green Publishing Company Assoc. and John Wiley Interscience, New York, 1989, 1992). It is appreciated to one skilled in the art that the polynucleotides of SEQ ID NOS:1-14 or fragments thereof (comprising at least 12 contiguous nucleotides), are particularly useful probes. Other particular useful probes for this purpose are hybridizable fragments to the sequences of SEQ ID NOS:1-14 (*i.e.*, comprising at least 12 contiguous nucleotides).

With respect to nucleic acid sequences which hybridize to specific nucleic acid sequences disclosed herein, hybridization may be carried out under conditions of reduced stringency, medium stringency or even stringent conditions. As an example of oligonucleotide hybridization, a polymer membrane containing immobilized denatured nucleic acids is first prehybridized for 30 minutes at 45°C in a solution consisting of 0.9 M NaCl, 50 mM NaH₂PO₄, pH 7.0, 5.0 mM Na₂EDTA, 0.5% SDS, 10X Denhardt's, and 0.5 mg/mL polyriboadenylic acid. Approximately 2 X 10⁷ cpm (specific activity 4-9 X 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30 minute wash in fresh 1X SET at Tm 10°C for the oligonucleotide probe. The membrane is then exposed to autoradiographic film for detection of hybridization signals.

Stringent conditions means hybridization will occur only if there is at least 90% identity, preferably at least 95% identity and most preferably at least 97% identity between the sequences. Further, it is understood that a section of a 100 bps sequence that is 95 bps in length has 95% identity with the 1090 bps sequence from which it is obtained. *See* J. Sambrook *et al*., *Molecular Cloning, A Laboratory Manual, 2d Ed*., Cold Spring Harbor Laboratory (1989) which is hereby incorporated by reference in its entirety. Also, it is understood that a fragment of a 100 bps sequence that is 95 bps in length has 95% identity with the 100 bps sequence from which it is obtained.

As used herein, a first DNA (RNA) sequence is at least 70% and preferably at least 80% identical to another DNA (RNA) sequence if there is at least 70% and preferably at least a 80% or 90% identity, respectively, between the bases of the first sequence and the bases of the another sequence, when properly aligned with each other, for example when aligned by BLASTN.

"Identity" as the term is used herein, refers to a polynucleotide sequence which comprises a percentage of the same bases as a reference polynucleotide (SEQ ID NOS:1-14). For example, a polynucleotide which is at least 90% identical to a reference polynucleotide, has polynucleotide bases which are identical in 90% of the bases which make up the reference polynucleotide and may have different bases in 10% of the bases which comprise that polynucleotide sequence.

The present invention relates polynucleotides which differ from the reference polynucleotide such that the changes are silent changes, for example the change do not alter the amino acid sequence encoded by the polynucleotide. The present invention also relates to nucleotide changes which result in amino acid substitutions, additions, deletions, fusions and truncations in the polypeptide encoded by the reference polynucleotide. In a preferred aspect of the invention these polypeptides retain the same biological action as the polypeptide encoded by the reference polynucleotide.

It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. The probes are thus useful to isolate complementary copies of DNA from other sources or to screen such sources for related sequences.

The polynucleotides of this invention were recovered from genomic gene libraries from the organisms listed in Table 1. For example, gene libraries can be generated in the Lambda ZAP II cloning vector (Stratagene Cloning Systems). Mass excisions can be performed on these libraries to generate libraries in the pBluescript phagemid. Libraries are thus generated and excisions performed according to the protocols/methods hereinafter described.

The excision libraries are introduced into the *E. coli* strain BW14893 F'kan1A. Expression clones are then identified using a high temperature filter assay. Expression clones encoding several glucanases and several other glycosidases are identified and repurified. The polynucleotides, and enzymes encoded thereby, of the present invention, yield the activities as described above.

The coding sequences for the enzymes of the present invention were identified by screening the genomic DNAs prepared for the clones having glucosidase or galactosidase activity.

An example of such an assay is a high temperature filter assay wherein expression clones were identified by use of high temperature filter assays using buffer Z (see recipe below) containing 1 mg/ml of the substrate 5-bromo-4-chloro-3-indolyl-β-D-glucopyranoside (XGLU) (Diagnostic Chemicals Limited or Sigma) after introducing an excision library into the *E. coli* strain BW14893 F'kan1A. Expression clones encoding XGLUases were identified and repurified from M11TL, OC1/4V, Pyrococcus furiosus VC1, Staphylothemus marinus F1, Thermococcus 9N-2, Thermotoga maritima MSB8, Thermococcus alcaliphilus AEDII12RA, and Thermococcus chitonophagus GC74.

Z-buffer: (referenced in Miller, J.H. (1992) A Short Course in Bacterial Genetics, p. 445.)
per liter:

| | |
|---|---|
| Na₂HPO₄-7H₂O | 16.1g |
| NaH₂PO₄-7H₂O | 5.5g |
| KCl | 0.75g |
| MgSO₄-7H₂O | 0.246g |
| β-mercaptoethanol | 2.7ml |
| Adjust pH to 7.0 | |

### High Temperature Filter Assay

(1) The f factor f'kan (from *E*. *coli* strain CSH118)(1) was introduced into the pho-pnh-lac-strain BW14893(2). BW13893(2). The filamentous phage library was plated on the resulting strain, BW14893 F'kan. (Miller, J.H. (1992) A Short Course in Bacterial Genetics; Lee, K.S., Metcalf, et al., (1992) Evidence for two phosphonate degradative pathways in Enterobacter Aerogenes, J. Bacteriol., 174:2501-2510.
(2) After growth on 100 mm LB plates containing 100 µg/ml ampicillin, 80 µg/ml nethicillin and 1mM IPTG, colony lifts were performed using Millipore HATF membrane filters.
(3) The colonies transferred to the filters were lysed with chloroform vapor in 150 mm glass petri dishes.
(4) The filters were transferred to 100 mm glass petri dishes containing a piece of Whatman 3MM filter paper saturated with buffer.
   (a) when testing for galactosidase activity (XGALase), 3MM paper was saturated with Z buffer containing 1 mg/ml XGAL (ChemBridge Corporation). After transferring filter bearing lysed colonies to the glass petri dish, placed dish in oven at 80-85°C.
   (b) when testing for glucosidase (XGLUase), 3MM paper was saturated with Z buffer containing 1 mg/ml XGLU. After transferring filter bearing lysed colonies to the glass petri dish, placed dish in oven at 80-85°C.
(5) 'Positives' were observed as blue spots on the filter membranes. Used the following filter rescue technique to retrieve plasmid from lysed positive colony. Used pasteur pipette (or glass capillary tube) to core blue spots on the filter membrane. Placed the small filter disk in an Eppendorf tube containing 20 µl water. Incubated the Eppendorf tube at 75°C for 5 minutes followed by vortexing to elute plasmid DNA off filter. This DNA was transformed into electrocompetent *E. coli* cells DH10B for Thermatoga maritima MSB8-6G, Staphylothermus marinus F1-12G, Thermococcus AEDII12RA-18B/G, Thermococcus chitonophagus GC74-22G, M11T1 and OC1/4V. Electrocompetent BW14893 F'kan1A *E*. *coli* were used for Thermococcus 9N2-31B/G, and *Pyrococcus furiosus* VC1-7G1. Repeated filter-lift assay on transformation plates to identify 'positives'. Return transformation plates to 37°C incubator after filter lift to regenerate colonies. Inoculate 3 ml LB liquid containing 100 µg/ml ampicillin with repurified positives and incubate at 37°C overnight. Isolate plasmid DNA from these cultures and sequence plasmid insert. In some instances where the plates used for the initial colony lifts contained non-confluent colonies, a specific colony corresponding to a blue spot on the filter could be identified on a regenerated plate and repurified directly, instead of using the filter rescue technique.

Another example of such an assay is a variation of the high temperature filter assay wherein colony-laden filters are heat-killed at different temperatures (for example, 105°C for 20 minutes) to monitor thermostability. The 3MM paper is saturated with different buffers (i.e., 100 mM NaCl, 5 mM MgCl₂, 100 mM Tris-Cl (pH 9.5)) to determine enzyme activity under different buffer conditions.

A β-glucosidase assay may also be employed, wherein GlcpβNp is used as an artificial substrate (aryl-β-glucosidase). The increase in absorbance at 405 nm as a result of p-nitrophenol (pNp) liberation was followed on a Hitachi U-1100 spectrophotometer, equipped with a thermostatted cuvette holder. The assays may be performed at 80°C or 90°C in closed 1-ml quartz cuvette. A standard reaction mixture contains 150 mM trisodium substrate, pH 5.0 (at 80°C), and 0.95 mM pNp derivative pNp = 0.561 mM⁻¹ • cm⁻¹). The reaction mixture is allowed to reach the desired temperature, after which the reaction is started by injecting an appropriate amount of enzyme (1.06 ml final volume).

1 U β-glucosidase activity is defined as that amount required to catalyze the formation of 1.0 µmol pNp/min. D-cellobiose may also be used as a substrate.

An ONPG assay for β-galactosidase activity is described by Miller, J.H. (1992) A Short Course in Bacterial Genetics and Mill, J.H. (1992) Experiments in Molecular Genetics, the contents of which are hereby incorporated by reference in their entirety.

A quantitative fluorometric assay for β-galactosidase specific activity is described by : Youngman P., (1987) Plasmid Vectors for Recovering and Exploiting Tn917 Transpositions in Bacillus and other Gram-Positive Bacteria. In Plasmids: A Practical approach (ed. K. Hardy) pp 79-103. IRL Press, Oxford. A description of the procedure can be found in Miller (1992) p. 75-77, the contents of which are incorporated by reference herein in their entirety.

The polynucleotides of the present invention may be in the form of DNA which DNA includes cDNA, genomic DNA, and synthetic DNA. The DNA may be double-stranded or single-stranded, and if single stranded may be the coding strand or non-coding (anti-sense) strand. The coding sequences which encodes the mature enzymes may be identical to the coding sequences shown in Figures 14 (SEQ ID NOS:14) or may be a different coding sequence which coding sequence, as a result of the redundancy or degeneracy of the genetic code, encodes the same mature enzymes as the DNA of Figures 14 (SEQ ID NO:14).

The polynucleotide which encodes for the mature enzyme of Figures 1-14 (SEQ ID NOS:15-28) may include, but is not limited to: only the coding sequence for the mature enzyme; the coding sequence for the mature enzyme and additional coding sequence such as a leader sequence or a proprotein sequence; the coding sequence for the mature enzyme (and optionally additional coding sequence) and non-coding sequence, such as introns or non-coding sequence 5' and/or 3' of the coding sequence for the mature enzyme.

Thus, the term "polynucleotide encoding an enzyme (protein)" encompasses a polynucleotide which includes only coding sequence for the enzyme as well as a polynucleotide which includes additional coding and/or non-coding sequence.

The present invention further relates to variants of the hereinabove described polynucleotides which encode for fragments, analogs and derivatives of the enzymes having the deduced amino acid sequences of Figures 1-14 (SEQ ID NOS:15-28). The variant of the polynucleotide may be a naturally occurring allelic variant of the polynucleotide or a non-naturally occurring variant of the polynucleotide.

Thus, the present invention includes polynucleotides encoding the same mature enzymes as shown in Figures 14 (SEQ ID 28) as well as variants of such polynucleotides which variants encode for a fragment, derivative or analog of the enzymes of Figures 1-14 (SEQ ID NOS:15-28). Such nucleotide variants include deletion variants, substitution variants and addition or insertion variants.

As hereinabove indicated, the polynucleotides may have a coding sequence which is a naturally occurring allelic variant of the coding sequences shown in Figures 1-14 (SEQ ID NOS:1-14). As known in the art, an allelic variant is an alternate form of a polynucleotide sequence which may have a substitution, deletion or addition of one or more nucleotides, which does not substantially alter the function of the encoded enzyme.

Fragments of the full length gene of the present invention may be used as a hybridization probe for a cDNA or a genomic library to isolate the full length DNA and to isolate other DNAs which have a high sequence similarity to the gene or similar biological activity. Probes of this type preferably have at least 10, preferably at least 15, and even more preferably at least 30 bases and may contain, for example, at least 50 or more bases. The probe may also be used to identify a DNA clone corresponding to a full length transcript and a genomic clone or clones that contain the complete gene including regulatory and promoter regions, exons, and introns. An example of a screen comprises isolating the coding region of the gene by using the known DNA sequence to synthesize an oligonucleotide probe. Labeled oligonucleotides having a sequence complementary to that of the gene of the present invention are used to screen a library of genomic DNA to determine which members of the library the probe hybridizes to.

The present invention further relates to polynucleotides which hybridize to the hereinabove-described sequences if there is at least 70%, preferably at least 90%, and more preferably at least 95% identity between the sequences. The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode enzymes which either retain substantially the same biological function or activity as the mature enzyme encoded by the DNA of Figures 1-14 (SEQ ID NOS:1-14).

Alternatively, the polynucleotide may have at least 15 bases, preferably at least 30 bases, and more preferably at least 50 bases which hybridize to any part of a polynucleotide of the present invention and which has an identity thereto, as hereinabove described, and which may or may not retain activity. For example, such polynucleotides may be employed as probes for the polynucleotides of SEQ ID NOS:1-14, for example, for recovery of the polynucleotide or as a diagnostic probe or as a PCR primer.

Thus, the present invention is directed to polynucleotides having at least a 70% identity, preferably at least 90% identity and more preferably at least a 95% identity to a polynucleotide which encodes the enzymes of SEQ ID NOS:28 as well as fragments thereof, which fragments have at least 15 bases, preferably at least 30 bases and most preferably at least 50 bases, which fragments are at least 90% identical, preferably at least 95% identical and most preferably at least 97% identical under stringent conditions to any portion of a polynucleotide of the present invention.

The present invention further relates to enzymes which have the deduced amino acid sequences of Figures 14 (SEQ ID NOS:28) as well as fragments, analogs and derivatives of such enzyme.

The terms "fragment," "derivative" and "analog" when referring to the enzymes of Figures 1-14 (SEQ ID NOS:15-28) means enzymes which retain essentially the same biological function or activity as such enzymes. Thus, an analog includes a proprotein which can be activated by cleavage of the proprotein portion to produce an active mature enzyme.

The enzymes of the present invention may be a recombinant enzyme, a natural enzyme or a synthetic enzyme, preferably a recombinant enzyme.

The fragment, derivative or analog of the enzymes of Figures 1-14 (SEQ ID NOS:15-28) may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature enzyme is fused with another compound, such as a compound to increase the half-life of the enzyme (for example, polyethylene glycol), or (iv) one in which the additional amino acids are fused to the mature enzyme, such as a leader or secretory sequence or a sequence which is employed for purification of the mature enzyme or a proprotein sequence. Such fragments, derivatives and analogs are deemed to be within the scope of those skilled in the art from the teachings herein.

The enzymes and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or enzyme present in a living animal is not isolated, but the same polynucleotide or enzyme, separated from some or all of the coexisting materials in the natural system, is isolated. Such polynucleotides could be part of a vector and/or such polynucleotides or enzymes could be part of a composition, and still be isolated in that such vector or composition is not part of its natural environment.

The enzymes of the present invention include the enzymes of SEQ ID NOS:28 (in particular the mature enzyme) as well as enzymes which have at least 70% similarity (preferably at least 70% identity) to the enzymes of SEQ ID NOS: 28 and more preferably at least 90% similarity (more preferably at least 90% identity) to the enzymes of SEQ ID NOS:15-28 and still more preferably at least 95% similarity (still more preferably at least 95% identity) to the enzymes of SEQ ID NOS: 28 and also include portions of such enzymes with such portion of the enzyme generally containing a= least 30 amino acids and more preferably at least 50 amino acids.

As known in the art "similarity" between two enzymes is determined by comparing the amino acid sequence and its conserved amino acid substitutes of one enzyme to the sequence of a second enzyme.

A variant, i.e. a "fragment", "analog" or "derivative" polypeptide, and reference polypeptide may differ in amino acid sequence by one or more substitutions, additions, deletions, fusions and truncations, which may be present in any combination.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Most highly preferred are variants which retain the same biological function and activity as the reference polypeptide from which it varies.

Fragments or portions of the enzymes of the present invention may be employed for producing the corresponding full-length enzyme by peptide synthesis; therefore, the fragments may be employed as intermediates for producing the full-length enzymes. Fragments or portions of the polynucleotides of the present invention may be used to synthesize full-length polynucleotides of the present invention.

The present invention also relates to vectors which include polynucleotides of the present invention, host cells which are genetically engineered with vectors of the invention and the production of enzymes of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention may be employed for producing enzymes by recombinant techniques. Thus, for example, the polynucleotide may be included in any one of a variety of expression vectors for expressing an enzyme. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Bacillus subtilis; fungal cells, such as yeast; insect cells such as Drosophila S2 and Spodoptera Sf9; animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example; Bacterial: pQE70, pQE60, pQE-9 (Qiagen), pD10, psiX174, pBluescript II KS, pNH8A, pNH16a, pNH18A, pNH46A (Stratagene); ptrc99a, pKK223-3, pKK233-3, pDR540, pRIT5 (Pharmacia); Eukaryotic: pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986) ) .

The constructs in host cells can be used in a conventional manner to produce the gene product encoded by the recombinant sequence. Alternatively, the enzymes of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the enzymes of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated enzyme. Optionally, the heterologous sequence can encode a fusion enzyme including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The enzyme can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The enzymes of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the enzymes of the present invention may be glycosylated or may be non-glycosylated. Enzymes of the invention may or may not also include an initial methionine amino acid residue.

β-galactosidase hydrolyzes lactose to galactose and glucose. Accordingly, the OC1/4V, 9N2-31B/G, AEDII12RA-18B/G and F1-12G enzymes may be employed in the food processing industry for the production of low lactose content milk and for the production of galactose or glucose from lactose contained in whey obtained in a large amount as a by-product in the production of cheese. Generally, it is desired that enzymes used in food processing, such as the aforementioned β-galactosidases, be stable at elevated temperatures to help prevent microbial contamination.

These enzymes may also be employed in the pharmaceutical industry. The enzymes are used to treat intolerance to lactose. In this case, a thermostable enzyme is desired, as well. Thermostable β-galactosidases also have uses in diagnostic applications, where they are employed as reporter molecules.

Glucosidases act on soluble cellooligosaccharides from the non-reducing end to give glucose as the sole product. Glucanases (endo- and exo-) act in the depolymerization of cellulose, generating more non-reducing ends (endo-glucanases, for instance, act on internal linkages yielding cellobiose, glucose and cellooligosaccharides as products). β-glucosidases are used in applications where glucose is the desired product. Accordingly, M11TL, F1-12G, GC74-22G and MSB8-6G (and OC1/4V, VC1-7G1, 9N2-31B/G and AEDII12RA18B/G) may be employed in a wide variety of industrial applications, including in corn wet milling for the separation of starch and gluten, in the fruit industry for clarification and equipment maintenance, in baking for viscosity reduction, in the textile industry for the processing of blue jeans, and in the detergent industry as an additive. For these and other applications, thermostable enzymes are desirable.

Antibodies generated against the enzymes corresponding to a sequence of the present invention can be obtained by direct injection of the enzymes into an animal or by administering the enzymes to an animal, preferably a nonhuman. The antibody so obtained will then bind the enzymes itself. In this manner, even a sequence encoding only a fragment of the enzymes can be used to generate antibodies binding the whole native enzymes. Such antibodies can then be used to isolate the enzyme from cells expressing that enzyme.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic enzyme products of this invention. Also, transgenic mice may be used to express humanized antibodies to immunogenic enzyme products of this invention.

Antibodies generated against the enzyme of the present invention may be used in screening for similar enzymes from other organisms and samples. Such screening techniques are known in the art, for example, one such screening assay is described in "Methods for Measuring Cellulase Activities", *Methods in enzymology,* Vol 160, pp. 87-116, which is hereby incorporated by reference in its entirety.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al*., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1

### Bacterial Expression and Purification of Glycosidase Enzymes

DNA encoding the enzymes of the present invention, SEQ ID 14, were initially amplified from a pBluescript vector containing the DNA by the PCR technique using the primers noted herein. The amplified sequences were then inserted into the respective PQE vector listed beneath the primer sequences, and the enzyme was expressed according to the protocols set forth herein. The 5' and 3' primer sequences for the respective genes are as follows:

Thermococcus AEDII12RA -18B/G
5'   CCGAGAATTCATTAAAGAGGAGAAATTAACTATGGTGAATGCTATGATTGTC
(SEQ ID NO:29)

3' CGGAAGATCTTCATAGCTCCGGAAGCCCATA (SEQ ID NO:30)
Vector: pQE12; and contains the following restriction enzyme
sites 5' EcoRI and 3' Blg II.

OC1/4V-33B/G
5'   CCGAGAATTCATTAAAGAGGAGAAATTAACTATGATAAGAAGGTCCGATTTTCC
(SEQ ID NO:31)

3′  CGGAAGATCTTTAAGATTTTAGAAATTCCTT (SEQ ID NO:32)
Vector: pQE12; and contains the following restriction enzyme
sites 5′ EcoRI and 3′ Bgl II.

Thermococcus 9N2 - 31B/G
5′ CCGAGAATTCATTAAAGAGGAGAAATTAACTATGCTACCAGAAGGCTTTCTC
(SEQ ID NO:33)

3'  CGGAGGTACCTCACCCAAGTCCGAACTTCTC (SEQ ID NO:34)
Vector:  pQE30; and contains the following restriction enzyme
sites 5' EcoRI and 3' KpnI.

Staphylothermus marinus F1 - 12G
5' CCGAGAATTCATTAAAGAGGAGAAATTAACTATGATAAGGTTTCCTGATTAT
(SEQ ID NO:35)

3' CGGAAGATCTTTATTCGAGGTTCTTTAATCC (SEQ ID NO:36)
Vector: pQE12; and contains the following restriction enzyme
sites 5' EcoRI and 3' Bgl II.

Thermococcus chitonophagus GC74 - 22G
5' CCGAGAATTCATTCATTAAAGAGGAGAAATTAACTATGCTTCCAGGAGAACTTTCTC
(SEQ ID NO:37)

3'  CGGAGGATCCCTACCCCTCCTCTAAGATCTC (SEQ ID NO:38)
Vector:  pQE12; and contains the following restriction enzyme
sites 5' EcoRI and 3' BamHI.

M11TL
5' AATAATCTAGAGCATGCAATTCCCCAAAGACTTCATGATAG (SEQ ID NO:39)

3' AATAAAAGCTTACTGGATCAGTGTAAGATGCT (SEQ ID NO:40)
Vector: pQE70; and contains the following restriction enzyme
sites 5' SphI and 3' Hind III.

Thermotoga maritima MSB8-6G
5′    CCGACAATTGATTAAAGAGGAGAAATTAACTATGGAAAGGATCGATGAAATT
(SEQ ID NO:41)

3'   CGGAGGTACCTCATGGTTTGAATCTCTTCTC (SEQ ID NO:42)
Vector:  pQE12; and contains the following restriction enzyme
sites 5' EcoRI and 3' KpnI.

Pyrococcus furiosus VC1 - 7G1
5'    CCGACAATTGATTAAAGAGGAGAAATTAACTATGTTCCCTGAAAAGTTCCTT
(SEQ ID NO:43)

3'   CGGAGGTACCTCATCCCCTCAGCAATTCCTC (SEQ ID NO:44)
Vector:  pQE12; and contains the following restriction enzyme
sites 5' EcoRI and 3' Kpn I.

Bankia gouldi endoglucanase (37GP1)
5'    AATAAGGATCCGTTTAGCGACGCTCGC
(SEQ ID NO:45)

3'    AATAAAAGCTTCCGGGTTGTACAGCGGTAATAGGC    (SEQ ID NO:46)
Vector:  pQE52; and contains the following restriction enzyme
sites 5' Bam HI and 3' Hind III.

Thermotoga maritima α-galactosidase (6GC2)
5′    TTTATTGAATTCATTAAAGAGGAGAAATTAACTATGATCTGTGTGGAAATATTCGGAAAG
(SEQ ID NO:47)

3'    TCTATAAAGCTTTCATTCTCTCTCACCCTCTTCGTAGAAG    (SEQ ID NO:48)

Vector:  pQET; and contains the following restriction enzyme
sites 5' EcoRI and 3' Hind III.

Thermotoga maritima  ß-mannanase (6GP2)
5'    TTTATTCAATTGATTAAAGAGGAGAAATTAACTATGGGGATTGGTGGCGACGAC
(SEQ ID NO:49)

3' TTTATTAAGCTTATCTTTTCATATTCACATACCTCC (SEQ ID NO:50)
Vector: pQEt; and contains the following restriction enzyme
sites 5' Hind III and 3' EcoRI.
AEPII 1a ß-mannanase (63GB1)

5'    TTTATTGAATTCATTAAAGAGGAGAAATTAACTATGCTACCAGAAGAGTTCCTATGGGGC
(SEQ ID NO:51)

3'    TTTATTAAGCTTCTCATCAACGGCTATGGTCTTCATTTC  (SEQ ID NO:52)
Vector:  pQEt; and contains the following restriction enzyme
sites 5' Hind III and 3' EcoRI.
OC1/4V  endoglucanase (33GP1)

5′ AAAAAACAATTGAATTCATTAAAGAGGAGAAATTAACTATGGTAGAAAGACACTTCAGATATGTTCTT
(SEQ ID NO:53)

3'    TTTTTCGGATCCAATTCTTCATTTACTCTTTGCCTG   (SEQ ID NO:54)
Vector:  pQEt; and contains the following restriction enzyme
sites 5' BamHI and 3' EcoRI.

Thermotoga maritima  pullalanase (6GP3)
5'    TTTTGGAATTCATTAAAGAGGAGAAATTAACTATGGAACTGATCATAGAAGGTTAC
(SEQ ID NO:55)

3'    ATAAGAAGCTTTTCACTCTCTGTACAGAACGTACGC  (SEQ ID NO:56)
Vector:  pQEt; and contains the following restriction enzyme
sites 5' EcoRI and 3' Hind III.

The restriction enzyme sites indicated correspond to the restriction enzyme sites on the bacterial expression vector indicated for the respective gene (Qiagen, Inc. Chatsworth, CA). The pQE vector encodes antibiotic resistance (Amp'), a bacterial origin of replication (ori), an IPTG-regulatable promoter operator (P/O), a ribosome binding site (RBS), a 6-His tag and restriction enzyme sites.

The pQE vector was digested with the restriction enzymes indicated. The amplified sequences were ligated into the respective pQE vector and inserted in frame with the sequence encoding for the RBS. The ligation mixture was then used to transform the E. coli strain M15/pREP4 (Qiagen, Inc.) by electroporation. M15/pREP4 contains multiple copies of the plasmid pREP4, which expresses the lacI repressor and also confers kanamycin resistance (Kan'). Transformants were identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture was used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation.

The primer sequences set out above may also be employed to isolate the target gene from the deposited material by hybridization techniques described above.

### Example 2

### Isolation of A Selected Clone From the Deposited genomic clones

A clone is isolated directly by screening the deposited material using the oligonucleotide primers set forth in Example 1 for the particular gene desired to be isolated. The specific oligonucleotides are synthesized using an Applied Biosystems DNA synthesizer. The oligonucleotides are labeled with ³²P- -ATP using T4 polynucleotide kinase and purified according to a standard protocol (Maniatis et al., Molecular Cloning: A Laboratory Manual, Cold Spring Harbor Press, Cold Spring, NY, 1982). The deposited clones in the pBluescript vectors may be employed to transform bacterial hosts which are then plated on 1.5% agar plates to the density of 20,000-50,000 pfu/150 mm plate. These plates are screened using Nylon membranes according to the standard screening protocol (Stratagene, 1993). Specifically, the Nylon membrane with denatured and fixed DNA is prehybridized in 6 x SSC, 20 mM NaH₂PO₄, 0.4%SDS, 5 x Denhardt's 500 µg/ml denatured, sonicated salmon sperm DNA; and 6 x SSC, 0.1% SDS. After one hour of prehybridization, the membrane is hybridized with hybridization buffer 6xSSC, 20 mM NaH₂PO₄, 0.4%SDS, 500 ug/ml denatured, sonicated salmon sperm DNA with 1x10⁶ cpm/ml ³²P-probe overnight at 42°C. The membrane is washed at 45-50°C with washing buffer 6 x SSC, 0.1% SDS for 20-30 minutes dried and exposed to Kodak X-ray film overnight. Positive clones are isolated and purified by secondary and tertiary screening. The purified clone is sequenced to verify its identity to the primer sequence.

Once the clone is isolated, the two oligonucleotide primers corresponding to the gene of interest are used to amplify the gene from the deposited material. A polymerase chain reaction is carried out in 25 µl of reaction mixture with 0.5 ug of the DNA of the gene of interest. The reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with the Perkin-Elmer Cetus automated thermal cycler. The amplified product is analyzed by agarose gel electrophoresis and the DNA band with expected molecular weight is excised and purified. The PCR product is verified to be the gene of interest by subcloning and sequencing the DNA product. The ends of the newly purified genes are nucleotide sequenced to identify full length sequences. Complete sequencing of full length genes is then performed by Exonuclease III digestion or primer walking.

### Example 3

### Screening for Galactosidase Activity

Screening procedures for α-galactosidase protein activity may be assayed for as follows:

Substrate plates were provided by a standard plating procedure. Dilute XL1-Blue MRF *E coli* host of (Stratagene Cloning Systems, La Jolla, CA) to O.D.₆₀₀ = 1.0 with NZY media. In 15 ml tubes, inoculate 200 µl diluted host cells with phage. Mix gently and incubate tubes at 37 °C for 15 min. Add approximately 3.5 ml LB top agarose (0.7%) containing 1mM IPTG to each tube and pour onto all NYZ plate surface. Allow to cool and incubate at 37 °C overnight. The assay plates are obtained as substrate p-Nitrophenyl α-galactosidase (Sigma) (200 mg/100 ml) (100 mM NaCl, 100 mM Potassium-Phosphate) 1% (w/v) agarose. The plaques are overlayed with nitrocellulose and incubated at 4 °C for 30 minutes whereupon the nitrocellulose is removed and overlayed onto the substrate plates. The substrate plates are then incubated at 70 °C for 20 minutes.

### Example 4

### Screening of Clones for Mannanase Activity

A solid phase screening assay was utilized as a primary screening method to test clones for β-mannanase activity.

A culture solution of the Y1090-*E. coli* host strain (Stratagene Cloning Systems, La Jolla, CA) was diluted to O.D.₆₀₀=1.0 with NZY media. The amplified library from *Thermotoga maritima* lambda gtll library was diluted in SM (phage dilution buffer): 5 x 10⁷ pfu/µl diluted 1:1000 then 1:100 to 5 x 10² pfu/µl. Then 8 µl of phage dilution (5 x 10² pfu/µl) was plated in 200 µl host cells. They were then incubated in 15 ml tubes at 37 °C for 15 minutes.

Approximately 4 ml of molten, LB top agarose (0.7%) at approximately 52 °C was added to each tube and the mixture was poured onto the surface of LB agar plates. The agar plates were then incubated at 37 °C for five hours. The plates were replicated and induced with 10 mM IPTG-soaked Duralon-UV™ nylon membranes (Stratagene Cloning Systems, La Jolla, CA) overnight. The nylon membranes and plates were marked with a needle to keep their orientation and the nylon membranes were then removed and stored at 4 °C.

An Azo-galactomannan overlay was applied to the LB plates containing the lambda plaques. The overlay contains 1% agarose, 50 mM potassium-phosphate buffer pH 7, 0.4% Azocarob-galactomannan. (Megazyme, Australia). The plates were incubated at 72 °C. The Azocarob-galactomannan treated plates were observed after 4 hours then returned to incubation overnight. Putative positives were identified by clearing zones on the Azocarob-galactomannan plates. Two positive clones were observed.

The nylon membranes referred to above, which correspond to the positive clones were retrieved, oriented over the plate and the portions matching the locations of the clearing zones for positive clones wre cut out. Phage was eluted from the membrane cut-out portions by soaking the individual portions in 500 µl SM (phage dilution buffer) and 25 µl CHCl₃.

### Example 5

### Screening of Clones for Mannosidase Activity

A solid phase screening assay was utilized as a primary screening method to test clones for β-mannosidase activity.

A culture solution of the Y1090-*E*. *coli* host strain (Stratagene Cloning Systems, La Jolla, CA) was diluted to O.D.₆₀₀=1.0 with NZY media. The amplified library from AEPII 1a lambda gtl1 library was diluted in SM (phage dilution buffer): 5 x 10⁷ pfu/µl diluted 1:1000 then 1:100 to 5 x 10² pfu/µl. Then 8 µl of phage dilution (5 x 10² pfu/µl) was plated in 200 µl host cells. They were then incubated in 15 ml tubes at 37 °C for 15 minutes.

Approximately 4 ml of molten, LB top agarose (0.7%) at approximately 52 °C was added to each tube and the mixture was poured onto the surface of LB agar plates. The agar plates were then incubated at 37 °C for five hours. The plates were replicated and induced with 10 mM IPTG-soaked Duralon-UV™ nylon membranes (Stratagene Cloning Systems, La Jolla, CA) overnight. The nylon membranes and plates were marked with a needle to keep their orientation and the nylon membranes were then removed and stored at 4 °C.

A p-nitrophenyl-β-D-manno-pyranoside overlay was applied to the LB plates containing the lambda plaques. The overlay contains 1% agarose, 50 mM potassium-phosphate buffer pH 7, 0.4% p-nitrophenyl-β-D-manno-pyranoside. (Megazyme, Australia). The plates were incubated at 72 °C. The p-nitrophenyl-β-D-manno-pyranoside treated plates were observed after 4 hours then returned to incubation overnight. Putative positives were identified by clearing zones on the p-nitrophenyl-β-D-manno-pyranoside plates. Two positive clones were observed.

The nylon membranes referred to above, which correspond to the positive clones were retrieved, oriented over the plate and the portions matching the locations of the clearing zones for positive clones wre cut out. Phage was eluted from the membrane cut-out portions by soaking the individual portions in 500 µl SM (phage dilution buffer) and 25 µl CHCl₃.

### Example 6

### Screening for Pullulanase Activity

Screening procedures for pullulanase protein activity may be assayed for as follows:

Substrate plates were provided by a standard plating procedure. Host cells are diluted to O.D.₆₀₀ = 1.0 with NZY or appropriate media. In 15 ml tubes, inoculate 200 µl diluted host cells with phage. Mix gently and incubate tubes at 37 °C for 15 min. Add approximately 3.5 ml LB top agarose (0.7%) is added to each tube and the mixture is plated, allowed to cool, and incubated at 37°C for about 28 hours. Overlays of 4.5 mls of the following substrate are poured:

| 100 ml total volume | |
|---|---|
| 0.5g | Red Pullulan Red (Megazyme, Australia) |
| 1.0g | Agarose |
| 5ml | Buffer (Tris-HCL pH 7.2 @ 75 °C) |
| 2ml | 5M NaCl |
| 5ml | CaCl₂ (100mM) |
| 85ml | dH₂O |

Plates are cooled at room temperature, and thenm incubated at 75°C for 2 hours. Positives are observed as showing substrate degradation.

### Example 7

### Screening for Endoglucanase Activity

Screening procedures for endoglucanase protein activity may be assayed for as follows:
1. The gene library is plated onto 6 LB/GelRite/0.1% CMC/NZY agar plates (∼4,800 plaque forming units/plate) in E.coli host with LB agarose as top agarose. The plates are incubated at 37°C overnight.
2. Plates are chilled at 4°C for one hour.
3. The plates are overlayed with Duralon membranes (Stratagene) at room temperature for one hour and the membranes are oriented and lifted off the plates and stored at 4°C.
4. The top agarose layer is removed and plates are incubated at 37°C for -3 hours.
5. The plate surface is rinsed with NaCl.
6. The plate is stained with 0.1% Congo Red for 15 minutes.
7. The plate is destained with 1M NaCl.
8. The putative positives identified on plate are isolated from the Duralon membrane (positives are identified by clearing zones around clones). The phage is eluted from the membrane by incubating in 500µl SM + 25µl CHCl₃ to elute.
9. Insert DNA is subcloned into any appropriate cloning vector and subclones are reassayed for CMCase activity using the following protocol:
   i) Spin 1ml overnight miniprep of clone at maximum speed for 3 minutes.
   ii) Decant the supernatant and use it to fill "wells" that have been made in an LB/GelRite/0.1% CMC plate.
   iii) Incubate at 37°C for 2 hours.
   iv) Stain with 0.1% Congo Red for 15 minutes.
   v) Destain with 1M NaCl for 15 minutes.
   vi) Identify positives by clearing zone around clone.

Numerous modifications and variations of the present invention are possible in light of the above teachings and, therefore, within the scope of the appended claims, the invention may be practiced otherwise than as particularly described.

## Claims

1. A polynucleotide selected from the group consisting of:
(a) a polynucleotide comprising a nucleic acid sequence consisting of the nucleic acid sequence shown in SEQ ID NO:14;
(b) a polynucleotide which encodes an enzyme comprising an amino acid sequence shown in SEQ ID NO: 28;
(c) a polynucleotide encoding an enzyme encoded by the DNA contained in ATCC Deposit No. 97379, wherein said enzyme is a pullulanase;
(d) a polynucleotide having at least 70%, 80%, 90%, 95% or 97% identity to a polynucleotide of (a), (b) or (c), or a polynucleotide that hybridizes under stringent conditions to SEQ ID NO:14, which encodes a polypeptide having pullulanase activity or an alpha glycosidase activity;
(e) a polynucleotide which is complementary to a polynucleotide of (a), (b), (c) or (d); and
(f) a polynucleotide comprising at least 15 bases of a polynucleotide of (a), (b) or (c).

2. The polynucleotide of claim 1, wherein the polynucleotide is DNA or RNA.

3. A vector comprising the polynucleotide of claim 1 or 2.

4. A host cell comprising the vector of claim 3.

5. A process for producing a polypeptide comprising expressing from the host cell of claim 4 a polypeptide encoded by said polynucleotide of claim 1, part (a), (b), (c) and (d).

6. A process for producing a cell comprising transforming or transfecting the cell with the vector of claim 3 such that the cell expresses the polypeptide encoded by the polynucleotide contained in the vector.

7. An enzyme having a pullulanase or an alpha glycosidase activity comprising a member selected from the group consisting of:
(a) an enzyme encoded by the polynucleotide of claim 1, parts (a) to (d);
(b) an enzyme comprising an amino acid sequence which is at least 70%, 90% or 95% identical to the amino acid sequence set forth in SEQ ID NO: 28, or an enzyme comprising an amino acid sequence that varies by at least one conservative substitution of the amino acid sequence set forth in SEQ ID NO: 28;
(c) an enzyme which comprises at least 30 amino acid residues of the enzyme of (a) or (b); and
(d) an enzyme obtainable by the process of claim 5.

8. An antibody directed against the enzyme of claim 7.

9. A pharmaceutical composition comprising the polynucleotide of claim 1 and/or the enzyme of claim 7 and optionally a pharmaceutically acceptable carrier and/or diluent.

10. A diagnostic composition comprising the polynucleotide of claim 1 and/or the enzyme of claim 7.

11. A method for generating glucose from soluble cellooligosaccharides comprising administering an effective amount of an enzyme having the amino acid sequence set forth in SEQ ID NO: 28 or as set forth in claim 7.

12. A method for the isolation of the enzyme of claim 7 comprising using the antibody of claim 10.

13. Use of the enzyme of claim 7 in corn wet milling for the separation of starch and gluten, in the fruit industry for clarification and equipment maintenance, in baking for viscosity reduction, in the textile industry for the processing of blue jeans and/or in the detergent industry as an additive.

14. A method for hydrolyzing starch comprising contacting a composition comprising a starch with an enzyme as set forth in claim 7.

15. A method for hydrolyzing an alpha 1,6-glucosidic linkage comprising contacting a polysaccharide comprising an alpha 1,6-glucosidic linkage with an enzyme as set forth in claim 7.

16. A method for making glucose or maltose from a starch comprising contacting a composition comprising a starch with an enzyme as set forth in claim 7 under conditions wherein the enzyme hydrolyzes the starch.

17. A method for making a sweetener comprising contacting a composition comprising a starch with an enzyme as set forth in claim 7 under conditions wherein the enzyme hydrolyzes the starch.

18. A method for treating a waste comprising contacting a waste comprising a starch or a composition comprising an alpha 1,6-glucosidic linkage with an enzyme as set forth in claim 7 under conditions wherein the enzyme can hydrolyze an alpha 1,6-glucosidic linkage.

19. A method for treating a textile comprising contacting the textile with an enzyme as set forth in claim 7 under conditions wherein the enzyme can hydrolyze a glycosidic linkage.

20. A method for treating an animal feed comprising contacting the animal feed with an enzyme as set forth in claim 7 under conditions wherein the enzyme can hydrolyze a glycosidic linkage.

21. A method for making a fuel comprising contacting a plant biomass with an enzyme as set forth in claim 7 under conditions wherein the enzyme can hydrolyze a glycosidic linkage.

22. A method for treating a baked product comprising contacting the baked product with an enzyme as set forth in claim 7 under conditions wherein the enzyme can hydrolyze a glycosidic linkage.

23. A method for separating starch and gluten comprising contacting a composition comprising a starch and a gluten with an enzyme as set forth in claim 7 under conditions wherein the enzyme can hydrolyze a glycosidic linkage.

24. A method for debranching a pullulan or a starch comprising contacting a composition comprising a starch and a pullulan with an enzyme as set forth in claim 7 under conditions wherein the enzyme can hydrolyze a glycosidic linkage.

25. A detergent comprising an enzyme as set forth in claim 7.

26. A textile comprising an enzyme as set forth in claim 7.

27. A feed comprising an enzyme as set forth in claim 7.

28. A plant biomass comprising an enzyme as set forth in claim 7.

29. A baked product comprising an enzyme as set forth in claim 7.

## Patentansprüche

1. Polynucleotid ausgewählt aus der Gruppe bestehend aus:
a) einem Polynucleotid, dass eine Nucleinsäure umfasst, die aus der in SEQ ID Nr. 14 gezeigten Nucleinsäuresequenz besteht;
b) einem Polynucleotid, das ein Enzym codiert, das eine in SEQ ID Nr. 28 gezeigte Aminosäuresequenz umfasst;
c) einem Polynucleotid, das ein Enzym codiert, das durch die in ATCC-Hinterlegungsnummer 97379 enthaltene DNA codiert wird, wobei das Enzym Pullulanase ist;
d) einem Polynucleotid mit mindestens 70 %, 80 %, 90 %, 95 % oder 97 % Identität zu einem Polynucleotid von a), b) oder c), oder einem Polynucleotid, das unter stringenten Bedingungen mit SEQ ID Nr. 14 hybridisiert, welches ein Polypeptid mit Pullulanaseaktivität oder α-Glycosidaseaktivität codiert;
e) einem Polynucleotid, das zu einem Polynucleotid von a), b), c) oder d) komplementär ist; und
f) einem Polynucleotid, das mindestens 15 Basen eines Polynucleotids von a), b) oder c) umfasst.

2. Polynucleotid nach Anspruch 1, wobei das Polynucleotid DNA oder RNA ist.

3. Vektor, der das Polynucleotid nach Anspruch 1 oder 2 umfasst.

4. Wirtszelle, die den Vektor nach Anspruch 3 umfasst.

5. Verfahren zur Herstellung eines Polynucleotids, umfassend das Exprimeren eines Polypeptids, das durch das Polynucleotid von Anspruch 1, Teil a), b), c) und d) codiert wird, in der Wirtszelle nach Anspruch 4.

6. Verfahren zur Herstellung einer Zelle, welches das Transformieren oder Transfizieren der Zelle mit dem Vektor nach Anspruch 3 umfasst, so dass die Zelle das Polypeptid exprimiert, das durch das im Vektor enthaltene Polynucleotid codiert wird.

7. Enzym mit Pullulanase- oder α-Glycosidaseactivität, umfassend ein Mitglied ausgewählt aus der Gruppe bestehend aus:
a) einem Enzym, das durch das Polynucleotid nach Anspruch 1, Teile a) bis d) codiert wird;
b) einem Enzym, umfassend eine Aminosäuresequenz, die mindestens 70%, 90% oder 95% Identität zu der in SEQ ID Nr. 28 angegebenen Aminosäuresequenz hat, oder ein Enzym, das eine Aminosäuresequenz umfasst, welche sich durch mindestens eine konservative Substitution von der in SEQ ID Nr. 28 gezeigten Aminosäuresequenz unterscheidet;
c) einem Enzym, das mindestens 30 Aminosäurereste des Enzyms aus a) oder b) umfasst; und
d) einem Enzym das durch das Verfahren nach Anspruch 5 erhältlich ist.

8. Antikörper gerichtet gegen das Enzym nach Anspruch 7.

9. Arzneimittel umfassend das Polynucleotid nach Anspruch 1 und/oder das Enzym nach Anspruch 7 und gegebenenfalls einen pharmazeutisch verträglichen Träger und/oder Verdünnungsmittel.

10. Diagnostische Zusammensetzung umfassend das Polynucleotid nach Anspruch 1 und/oder das Enzym nach Anspruch 7.

11. Verfahren zur Erzeugung von Glucose aus löslichen Cellooligosacchariden, umfassend das Verabreichen einer wirksamen Menge eines Enzyms mit der Aminosäuresequenz wie in SEQ ID Nr. 28 oder wie in Anspruch 7 angegeben.

12. Verfahren zur Isolierung des Enzyms nach Anspruch 7, umfassend die Verwendung des Antikörpers nach Anspruch 10.

13. Verwendung des Enzyms nach Anspruch 7 beim Nassmahlen von Korn zur Trennung von Stärke und Gluten, in der Früchteindustrie zur Klärung und Wartung der Geräte, beim Backen zur Reduzierung der Viskosität, in der Textilindustrie zur Verarbeitung von Bluejeans und/oder in der Detergensindustrie als ein Additiv.

14. Verfahren zum Hydrolysieren von Stärke, umfassend das Inkontaktbringen einer Stärke umfassenden Zusammensetzung mit einem Enzym nach Anspruch 7.

15. Verfahren zum Hydrolysieren einer α-1,6-glucosidischen Bindung, umfassend das Inkontaktbringen eines Polysaccharids, das eine α-1,6-glucosidische Bindung umfasst, mit einem Enzym nach Anspruch 7.

16. Verfahren zur Herstellung von Glucose oder Maltose aus Stärke, umfassend das Inkontaktbringen einer Stärke enthaltenden Zusammensetzung mit einem Enzym nach Anspruch 7 unter Bedingungen, bei denen das Enzym die Stärke hydrolysiert.

17. Verfahren zur Herstellung eines Süßstoffs, umfassend das Inkontaktbringen einer Stärke enthaltenden Zusammensetzung mit einem Enzym nach Anspruch 7 unter Bedingungen, bei denen das Enzym die Stärke hydrolysiert.

18. Verfahren zur Behandlung von Abfällen, umfassend das Inkontaktbringen des Stärke enthaltenden Abfalls oder einer Zusammensetzung, die eine α-1,6-glucosidische Bindung enthält, mit einem Enzym nach Anspruch 7 unter Bedingungen, bei denen das Enzym eine α-1,6-glucosidische Bindung hydrolysieren kann.

19. Verfahren zur Behandlung von Textilien, umfassend das Inkontaktbringen der Textilien mit einem Enzym nach Anspruch 7 unter Bedingungen, bei denen das Enzym eine glycosidische Bindung hydrolysieren kann.

20. Verfahren zur Behandlung von Tierfutter, umfassend das Inkontaktbringen des Tierfutters mit einem Enzym nach Anspruch 7 unter Bedingungen, bei denen das Enzym eine glycosidische Bindung hydrolysieren kann.

21. Verfahren zur Herstellung eines Brennstoffs, umfassend das Inkontaktbringen einer Pflanzenbiomasse mit einem Enzym nach Anspruch 7 unter Bedingungen, bei denen das Enzym eine glycosidische Bindung hydrolysieren kann.

22. Verfahren zur Behandlung eines Backerzeugnisses, umfassend das Inkontaktbringen des Backerzeugnisses mit einem Enzym nach Anspruch 7 unter Bedingungen, bei denen das Enzym eine glycosidische Bindung hydrolysieren kann.

23. Verfahren zu Trennung von Stärke und Gluten, umfassend das Inkontaktbringen einer Zusammensetzung, die Stärke und Gluten umfasst, mit einem Enzym nach Anspruch 7 unter Bedingungen, bei denen das Enzym eine glycosidische Bindung hydrolysieren kann.

24. Verfahren zur Entfernung von Verzweigungen eines Pullulans oder einer Stärke, umfassend das Inkontaktbringen einer Zusammensetzung, die eine Stärke oder ein Pullulan umfasst, mit einem Enzym nach Anspruch 7 unter Bedingungen, bei denen das Enzym eine glycosidische Bindung hydrolysieren kann.

25. Detergens, umfassend ein Enzym nach Anspruch 7.

26. Textilie, umfassend ein Enzym nach Anspruch 7.

27. Futtermittel, umfassend ein Enzym nach Anspruch 7.

28. Pflanzenbiomasse, umfassend ein Enzym nach Anspruch 7.

29. Backerzeugnis, umfassend ein Enzym nach Anspruch 7.

## Revendications

1. Polynucléotide choisi dans le groupe constitué de :
(a) un polynucléotide comprenant une séquence d'acide nucléique consistant en la séquence d'acide nucléique présentée dans SEQ ID NO : 14;
(b) un polynucléotide qui code une enzyme comprenant une séquence d'acide aminé présentée dans SEQ ID NO : 28 ;
(c) un polynucléotide qui code une enzyme codée par l'ADN contenu dans le dépôt ATCC No. 97379, ladite enzyme étant une pullulanase ;
(d) un polynucléotide ayant au moins 70%, 80%, 90%, 95% ou 97% d'identité avec un polynucléotide selon (a), (b) ou (c), ou un polynucléotide qui hybride dans des conditions stringentes à SEQ ID NO : 14, qui code un polypeptide ayant une activité pullulanase ou une activité alpha glycosidase ;
(e) un polynucléotide complémentaire d'un polynucléotide selon (a), (b), (c) ou (d); et
(f) un polynucléotide comprenant au moins 15 bases d'un polynucléotide selon (a), (b) ou (c).

2. Polynucléotide selon la revendication 1, ledit polynucléotide étant un ADN ou un ARN.

3. Vecteur comprenant le polynucléotide selon la revendication 1 ou 2.

4. Cellule hôte comprenant le vecteur selon la revendication 3.

5. Procédé pour produire un polypeptide, comprenant l'expression à partir de la cellule hôte selon la revendication 4 d'un polypeptide codé par ledit polynucléotide selon la revendication 1, partie (a), (b), (c) et (d).

6. Procédé pour produire une cellule, comprenant la transformation ou la transfection de la cellule avec le vecteur selon la revendication 3 de telle sorte que la cellule exprime le polypeptide codé par le polynucléotide contenu dans le vecteur.

7. Enzyme ayant une activité pullulanase ou une activité alpha glycosidase, comprenant un membre choisi dans le groupe constitué de :
(a) une enzyme codée par le polynucléotide de la revendication 1, parties (a) à (d) ;
(b) une enzyme comprenant une séquence d'acide aminé qui est au moins 70%, 90% or 95% identique à la séquence d'acide aminé présentée dans SEQ ID NO : 28, ou une enzyme comprenant une séquence d'acide aminé qui varie par au moins une substitution conservatrice de la séquence d'acide aminé présentée dans SEQ ID NO : 28 ;
(c) une enzyme qui comprend au moins 30 résidus d'acide aminé de l'enzyme (a) ou (b) ; et
(d) une enzyme susceptible d'être obtenue par le procédé de la revendication 5.

8. Anticorps dirigé contre l'enzyme selon la revendication 7.

9. Composition pharmaceutique comprenant le polynucléotide selon la revendication 1 et/ou l'enzyme selon la revendication 7 et, éventuellement, un support et/ou diluant acceptable sur le plan pharmaceutique.

10. Composition diagnostique comprenant le polynucléotide selon la revendication 1 et/ou l'enzyme selon la revendication 7.

11. Méthode pour générer du glucose à partir de cellooligosaccharides solubles comprenant l'administration d'une quantité efficace d'une enzyme ayant la séquence d'acide aminé présentée dans SEQ ID NO : 28 ou telle que présentée dans la revendication 7.

12. Méthode pour isoler l'enzyme selon la revendication 7, comprenant l'utilisation de l'anticorps de la revendication 10.

13. Utilisation de l'enzyme selon la revendication 7 dans le concassage à mouture humide du grain pour la séparation de l'amidon et du gluten, dans l'industrie du fruit pour la clarification et pour la maintenance des équipements, en cuisson pour réduire la viscosité, dans l'industrie textile pour le traitement de blue jeans et/ou dans l'industrie des détergents en tant qu'additif.

14. Procédé pour hydrolyser l'amidon, comprenant la mise en contact d'une composition comprenant un amidon avec une enzyme telle que décrite dans la revendication 7.

15. Procédé pour hydrolyser une liaison alpha 1,6-glucosidique, comprenant la mise en contact d'un polysaccharide comprenant une liaison alpha 1,6-glucosidique avec une enzyme telle que décrite dans la revendication 7.

16. Procédé pour fabriquer du glucose ou du maltose à partir d'un amidon, comprenant la mise en contact d'une composition comprenant un amidon avec une enzyme telle que décrite dans la revendication 7, dans des conditions dans lesquelles l'enzyme hydrolyse l'amidon.

17. Procédé pour fabriquer un édulcorant, comprenant la mise en contact d'une composition comprenant un amidon avec une enzyme telle que décrite dans la revendication 7, dans des conditions dans lesquelles l'enzyme hydrolyse l'amidon.

18. Procédé pour traiter un déchet, comprenant la mise en contact d'un déchet comprenant un amidon ou une composition comprenant une liaison alpha 1,6-glucosidique avec une enzyme telle que décrite dans la revendication 7 dans des conditions dans lesquelles l'enzyme peut hydrolyser une liaison alpha 1,6-glucosidique.

19. Procédé pour traiter un textile comprenant la mise en contact du textile avec une enzyme telle que décrite dans la revendication 7 dans des conditions dans lesquelles l'enzyme peut hydrolyser une liaison glycosidique.

20. Procédé pour traiter un aliment pour animaux comprenant la mise en contact de l'aliment pour animaux avec une enzyme telle que décrite dans la revendication 7 dans des conditions dans lesquelles l'enzyme peut hydrolyser une liaison glycosidique.

21. Procédé pour faire un carburant comprenant la mise en contact d'une biomasse de plante avec une enzyme telle que décrite dans la revendication 7 dans des conditions dans lesquelles l'enzyme peut hydrolyser une liaison glycosidique.

22. Procédé pour traiter un produit de boulangerie ou pâtisserie, comprenant la mise en contact du produit de boulangerie ou pâtisserie avec une enzyme telle que décrite dans la revendication 7 dans des conditions dans lesquelles l'enzyme peut hydrolyser une liaison glycosidique.

23. Procédé pour séparer l'amidon et le gluten comprenant la mise en contact d'une composition comprenant un amidon et un gluten avec une enzyme telle que décrite dans la revendication 7 dans des conditions dans lesquelles l'enzyme peut hydrolyser une liaison glycosidique.

24. Procédé pour dé-ramifier un pullulane ou un amidon comprenant la mise en contact d'une composition comprenant un amidon et un pullulane avec une enzyme telle que décrite dans la revendication 7 dans des conditions dans lesquelles l'enzyme peut hydrolyser une liaison glycosidique.

25. Détergent comprenant une enzyme telle que décrite dans la revendication 7.

26. Textile comprenant une enzyme telle que décrite dans la revendication 7.

27. Aliment comprenant une enzyme telle que décrite dans la revendication 7.

28. Biomasse de plante comprenant une enzyme telle que décrite dans la revendication 7.

29. Produit de boulangerie ou pâtisserie comprenant une enzyme telle que décrite dans la revendication 7.
